(19) **Europäisches Patentamt**
**European Patent Office**
**Office européen des brevets**

(11) **EP 1 064 532 B1**

(12) **EUROPEAN PATENT SPECIFICATION**

(45) Date of publication and mention
of the grant of the patent:
**17.03.2010 Bulletin 2010/11**

(51) Int Cl.:
***G01N 27/22*** *(2006.01)* ***G01N 27/07*** *(2006.01)*

(21) Application number: **99910644.6**

(22) Date of filing: **18.03.1999**

(86) International application number:
**PCT/IL1999/000156**

(87) International publication number:
**WO 1999/047907 (23.09.1999 Gazette 1999/38)**

(54) **DEVICE FOR THE DETERMINATION OF BLOOD CLOTTING BY RESISTANCE**

GERÄT ZUR BESTIMMUNG DER BLUT-KOAGULATION mittels Widerstandsmessungen

DISPOSITIF DE DETERMINATION DE LA COAGULATION DU SANG à partir de la RESISTANCE

(84) Designated Contracting States:
**DE ES FR GB IT SE**

(30) Priority: **19.03.1998 IL 12375798**
**19.06.1998 US 100108**
**23.10.1998 IL 12673898**

(43) Date of publication of application:
**03.01.2001 Bulletin 2001/01**

(73) Proprietor: **Inverness Medical Switzerland GmbH**
**6300 Zug (CH)**

(72) Inventors:
• **ROSEMBERG, Yossef**
**43305 Raanana (IL)**
• **FISH, Falk**
**69497 Tel Aviv (IL)**

• **LEVI, Raphael**
**56478 Yehud (IL)**

(74) Representative: **Bausch, Thorsten**
**Hoffmann - Eitle**
**Patent- und Rechtsanwälte**
**Arabellastrasse 4**
**81925 München (DE)**

(56) References cited:
| | |
|---|---|
| **EP-A2- 0 164 473** | **WO-A-95/14224** |
| **US-A- 3 674 012** | **US-A- 3 674 012** |
| **US-A- 3 699 437** | **US-A- 3 699 437** |
| **US-A- 3 700 408** | **US-A- 4 301 412** |
| **US-A- 5 269 175** | **US-A- 5 601 995** |
| **US-A- 5 628 961** | |

**Description**

FIELD AND BACKGROUND OF THE INVENTION

The present invention relates to a device and method for determining viscosity of a liquid sample by resistance, and, in particular, to a device and method for analyzing clotting of a blood specimen by a patient in a home testing environment, substantially without the need for intervention by highly trained clinical laboratory technicians or other technical medical personnel.

Clotting time is the amount of time required for a blood sample from a patient to clot. The determination of clotting time is important for patients who are receiving anti-coagulant therapy, such as those patients taking the drug warfarin, for example. The dosing regimen for such drugs must be determined for each individual patient, and then adjusted according to the clotting time. A dosing regimen which does not increase clotting time in one patient might result in an excessively and dangerously prolonged clotting time in another patient. The determination of dosing regimen is further complicated by the pharmacological properties of some anti-coagulant drugs, such as warfarin, which may remain highly bound to proteins in the blood, so that the amount of bioavailable drug in the body may be difficult to adjust. Thus, clotting time must be carefully monitored for each patient.

Unfortunately, currently clotting time can only be measured in a clinical laboratory setting by a clinical laboratory technician. The devices employed for the measurement of clotting time in the laboratory require the time period for clotting to be determined by optical or mechanical analysis of the blood sample. For example, a commercially available mechanical diagnostic assay is available from Boehringer Mannheim Corp and is based on the automated analysis of the behavior of metal balls which are stirred inside the plasma specimen by a magnet. These balls are examined with automated machine vision techniques to determine when the balls no longer move under the influence of the magnetic force. Clotting is assumed to have occurred when such lack of movement within the sample is observed. According to other disclosed diagnostic assays in the prior art, the blood sample to be analyzed is placed in a capillary. When the sample no longer moves through the capillary, clotting has occurred. The determination of the cessation of movement through the capillary is done by optical analysis. Optical analysis may be performed manually through observation of the sample within the capillary by a trained laboratory technician, or automatically by determining changes in the optical density of the blood sample with a light source and a photodetector.

For example, an optical device for manual visual detection of clotting has been disclosed in U.S. Patent No. 3,951,606. A similar, disposable device has been disclosed in PCT Application No. WO 96/00395. More automated devices, in which changes in optical density are measured by sending a beam of light through the sample and detecting the amount of light which passes through the sample by a photodetector, are disclosed in U.S. Patent No. 5,039,617 and U.S. Patent No. 5,197,017. All of these devices suffer from the drawback of being either complex to operate or complex to construct. For example, measurement of clotting time by visual inspection of a blood sample as it moves through a capillary requires intense concentration in order to accurately determine the moment when the sample stops moving. Such concentration may not be difficult for a trained laboratory technician, but places an excessive burden on the lay patient who wishes to measure clotting time in a home testing environment. Thus, although at least one disposable version of a device for determination of clotting time by visual analysis has been disclosed in PCT Application No. WO 96/00395, as yet no such device is available for the home testing market.

The alternative form for optical analysis, in which a beam of light is transmitted from a light source through the blood sample to a photodetector, suffers from the drawback of being too complex and expensive to manufacture as a home testing device. Such a device would also require careful maintenance and calibration on a regular basis, which also places an excessive burden on the lay patient for home testing. Thus, automated optical analysis is not suitable for the home testing environment.

Devices for the electrical determination of blood clotting have also been disclosed. For example, one such device determined clotting of a blood sample by measuring changes in the electrical impedance of the sample (A. Ur, Nature, 226:269-270, 1970 and US Patent No. 3,699,437). A similar device was also disclosed in U.S. Patent No. 3,674,012. However, this device required complex, delicate and sensitive laboratory equipment to measure the impedance of the sample, which would not be suitable for home testing. Furthermore, although the device was first disclosed more than twenty years ago, no commercially available equivalent device has been produced for the laboratory or for the home testing environment, an indication of the technical difficulties inherent in the design, production and operation of such a device. Thus, the measurement of electrical impedance is not suitable for the determination of clotting time in the home testing environment.

Other devices employing some type of electrical measurement of clotting time have been disclosed, but again none of these devices is suitable for the home testing environment. For example, U.S. Patent No. 5,491,408 discloses a device which determines changes in viscosity of a liquid sample by measuring variations in voltage as the sample is agitated by a vibration generator. Clearly, such a device would be sensitive to vibrations in the environment and would therefore not be suitable for operation by a lay patient.

DDR (former East German) Patent No. DD 237454 discloses an apparatus for determining blood coagulation time. One electrode is dipped at intervals into a blood sample held in a metal block thermostat and then raised, and the capacitance between the raised electrode and the metal block is measured. As the blood coagulates, a filament of viscous liquid is attached to the raised electrode, changing the capacitance. This apparatus would certainly not be suitable for operation by a lay patient, since it requires careful manipulation of the equipment and manual monitoring of the progress of coagulation. Furthermore, the disclosed apparatus could not easily be automated in a small portable device. Thus, the apparatus disclosed in DDR Patent No. DD 237454 would certainly not be suitable for the home testing environment.

As another example, U.S. Patent No. 5,601,995 discloses a device for detecting blood clotting. This device includes a porous sheet for receiving the blood sample. Clotting is then detected either optically or electrically, by the measurement of resistance. However, the porous sheet has a number of drawbacks as a receptacle for the blood sample. For example, various substances such as gelatin coating, surfactants or hydrophilic polymers are added to the sheet in order to overcome such problems as controlling the speed of transport through the porous medium and/or to promote adhesion of the fibrin clots (column 5, lines 16-34). In addition, since the blood (or any liquid specimen) moves inside the porous sheet, contact of the liquid with the electrodes, which touch the surface of the sheet, may not be sufficiently intimate to provide an accurate measurement of the electrical properties of the liquid specimen. Thus, the device disclosed in U.S. Patent No. 5,601,995 is clearly deficient.

As yet another example, both U.S. Patent No. 3,840,806 and PCT Application No. WO 94/16095 disclose devices which both measure electrical resistance and optical density of a sample. As noted previously, the measurement of optical density requires excessively complex equipment for the home testing environment. Furthermore, although over twenty years have passed since U.S. Patent No. 3,840,806 was publicly disclosed, and four years have passed since PCT Application No. WO 94/16095 was publicly disclosed, no such device has been made available for the home testing environment, nor has any announcement been made of any clinical trials for such a device. Thus, clearly these prior art electrical devices have not proven to be suitable for operation by the lay patient.

Finally, US Patent No. 5,628,961 discloses apparatus for conducting coagulation assays based on electrical measurements. The apparatus comprises a disposable, single-use cartridge connected to an external pump means capable of exerting a force against a fluid sample to move the sample within the housing. The apparatus is adapted for the point-of-care clinical diagnostic area, including use in accident sites, emergency rooms, surgery or intensive care units.

One example of a device which has been successfully used by the lay patient in the home testing environment can be found in an entirely different medical area. Diabetics now use a small, highly portable electrical device for the determination of blood glucose levels. The operation of this device is quite simple. A drop of blood is taken from the patient and placed on a small card which is inserted into the device. After a brief period of time, the concentration of glucose in the sample is displayed on a small screen. Thus, commercially available glucose testing devices are simple to operate by the lay patient and are sufficiently robust to withstand the rigors of the home testing environment, yet are sufficiently accurate to generate clinically significant results.

Clearly, an equivalent device for the determination of clotting time in the home environment by the lay patient would be highly useful and commercially successful. Home testing of clotting time offers a number of advantages, including greater clinical utility of measurement through daily assessments rather than by a weekly or bi-weekly measurement at a clinic, and greater convenience for the lay patient, enabling the patient to immediately seek medical attention in response to any sudden changes in the measured clotting time. Currently, patients must travel to a clinic, have a blood sample withdrawn by a healthcare professional, and then wait for the results. Such a complicated, inconvenient and time-consuming process can potentially result in reduced patient compliance. By contrast, testing in the home is potentially both more convenient and less expensive, and may also result in increased patient compliance. Unfortunately, such a home testing device for the measurement of clotting time is simply not available. Thus, lay patients cannot currently measure clotting time in the home, but must instead resort to the less desirable process of clinical testing.

There is therefore a need for, and it would be useful to have, a method and a device for the measurement of clotting time which can be performed by the lay patient in the home testing environment, which would be both robust and accurate, which would also be simple to operate and which could potentially be employed to determine the viscosity of other types of liquid samples, preferably through the measurement of electrical capacitance, and which is substantially automated for relatively minimal intervention by a lay patient.

SUMMARY OF THE INVENTION

The present invention provides a novel kit and method for the determination of the clotting time of a blood sample, as recited in claims 9 and 1.
wherein the blood sample undergoes an electrical analysis based upon the movement of the sample through a capillary channel, such that the distance traveled by the sample through the channel is proportional to the clotting time.

The electrical analysis is performed, additionally or alternatively, by measuring current passing between two electrodes, one on either side of the channel, such that the amount of current is proportional to the distance traveled by the sample.

In this way the present invention provides a device for the determination of clotting time in a format which is suitable for operation by a lay patient in the home testing environment.

These and other objects of the present invention will become apparent from the figures, description and claims.

Preferred embodiments are described in the dependent claims.

In particular, the test portion of the sample of the liquid enters the test chamber from a capillary force induced by a capillary structure of the test chamber.

Preferably, the capillary structure is selected from the group consisting of a capillary depth of the test chamber, and a narrow channel shape of the test chamber.

Preferably, the sample of the liquid is selected from the group consisting of whole blood, plasma and serum. More preferably, the sample of the liquid is whole blood. Most preferably, a change in the viscosity of the whole blood is caused by coagulation, such that a volume of the test chamber being occupied by the test portion of the whole blood is determined according to the coagulation. Even more preferably, the test chamber features at least one coagulant agent being in contact with the test portion of the whole blood, the at least one coagulant agent altering a clotting property of the test portion of the whole blood. Also most preferably, the coagulant agent is an anti-coagulant, such that the test portion of the whole blood clots slower than the reference portion of the whole blood. Alternatively and most preferably, the coagulant agent is a pro-coagulating factor, such that the test portion of the whole blood clots faster than the reference portion of the whole blood.

Preferably, the at least one coagulant agent is dried onto the test chamber. Alternatively and preferably, the at least one coagulant agent is a liquid droplet placed onto the test chamber.

Alternatively and preferably, the coagulant agent is a pro-coagulating factor, such that the reference portion of the whole blood clots faster than the test portion of the whole blood. Preferably, the at least one coagulant agent is dried onto the disposable sensor. Alternatively and preferably, the at least one coagulant agent is a liquid droplet placed onto the disposable sensor.

Preferably, the clotting time is determined as a prothrombin time. Alternatively and preferably, the clotting time is determined as a partial prothrombin time.

Preferably, the electrodes are constructed from a material selected from the group consisting of carbon, graphite and metal. More preferably, the material is a carbon, graphite or metal impregnated film. Alternatively and more preferably, the metal is in a form selected from the group consisting of film, foil, metal impregnated film and metal sputtered film. Most preferably, the metal is selected from the group consisting of silver, aluminum, titanium, stainless steel, palladium, copper and a mixture of silver and silver chloride.

According to yet other preferred embodiments, the electrodes extend along substantially the entirety of the test chamber. Preferably, the electrodes are disposed on opposing sides of the test chamber. More preferably, the electrodes extend along a portion of the test chamber, a length of the portion being shorter than a length of the test chamber.

According to still other preferred embodiments, the chamber features a plurality of surfaces, and the plurality of surfaces is pretreated. Preferably, the pretreated surfaces are pretreated by being soaked in a buffer containing an ingredient selected from the group consisting of a detergent and a hydrophilic polymer.

According to yet other preferred embodiments, the device further features a cover for covering said test chamber, said cover featuring at least one aperture. More preferably, the cover features a plurality of apertures arranged as a mesh.

According to still another embodiment of the present invention, there is provided a kit for measuring a prothrombin time of a blood sample of blood from a patient.

Preferably, the resistance is determined indirectly by measuring an amount of a current passing between the electrodes, such that the current is a measured current. More preferably, the clotting time is measured by determining the amount of the current after a predetermined period of time has elapsed. Alternatively and more preferably, the clotting time is measured by determining a period of time required for the amount of the current to reach a predetermined level.

Hereinafter, the term "subject" refers to the human or lower mammal upon which the method and system of the present invention are performed or operated. Hereinafter, the term "patient" refers to any human from which a blood sample may be drawn for analysis by the device and method of the present invention. Hereinafter, the terms "self-testing" and "self-operating" refer to the analysis of a blood sample from a patient either by the same patient or by another lay individual who is not a medical professional. Hereinafter, the terms "home testing environment" and "home environment" refer to an environment other than a clinic, clinical or research laboratory, office of a medical professional or hospital. An example of a home testing environment would be the home of a patient.

Hereinafter, the term "clotting time" includes the measurement of any parameter related to the time period required for a blood sample to clot. Examples of these parameters include, but are not limited, prothrombin time and partial prothrombin time. Hereinafter, the term "blood sample" may include, but is not limited to, a sample of whole blood, plasma or serum.

Hereinafter, the term "electrode" refers to any conductor which is capable of conducting electricity and is suitable for the measurement of electrical capacitance.

BRIEF DESCRIPTION OF THE DRAWINGS

The foregoing and other objects, aspects and advantages will be better understood from the following detailed description of a preferred embodiment of the invention with reference to the drawings, wherein:

FIG. 1 shows a flow chart of an exemplary method;
FIGS. 2A-2D are schematic block diagrams of various embodiments of exemplary devices for the determination of clotting time;
FIG. 3 is a schematic block diagram of another embodiment, which features apertures in the cover of the chambers of the sensor;
FIG. 4 is a flow chart of an exemplary method for determining the viscosity of a liquid through the measurement of resistance or current according to the present invention;
FIGS. 5A-5C are schematic block diagrams of exemplary devices for the determination of clotting time by resistance according to the present invention;
FIGS. 6A-6C show the effect of the addition of coagulant to blood samples on the measurement of current by a device of the present invention, in which Figure 6A shows the time to the maximum current, Figure 6B is the measured current after a fixed time period has elapsed and Figure 6C shows the corrected measured current;
FIGS. 7A and 7B show the variation of the current for three different blood samples from patients undergoing anticoagulant therapy;
FIG. 8 shows yet another embodiment of the device of the present invention, which features a mesh for the cover of the chambers of the sensor;
FIG. 9 shows a schematic block diagram of the circuit for testing the device of Figure 8;
FIGS. 10A and 10B show examples of transients for different INR values for the device of Figures 8 and 9; and
FIGS. 11A-11C depict the correlation between the clotting time values, as obtained with the sensor strip of the invention, and the INR values, as determined by the reference method.

DETAILED DESCRIPTION OF PREFERRED EMBODIMENTS

The present invention is directed to a method and a device for the measurement of the clotting time of a blood sample, especially in the home testing environment. The device of the present invention includes a body which is intended to receive a disposable card, or other disposable sensor, on which a blood sample is placed. The sensor is inserted into the body of the device, which then measures an electrical property, such as the amplitude of current passed through the sample is used to determine clotting time. Preferably, the determined clotting time is then displayed on a screen, for example.

The electrical property could be determined by placing two electrodes on either side of a test chamber. The distance traveled by the liquid sample through the chamber, such as a blood sample before clotting, would be directly related to the viscosity of the liquid. Also alternatively, the time period required for the maximum current to be reached could be measured, or the amount of current could be measured after a fixed time period had elapsed.

According to one preferred embodiment of the present invention, the electrodes could be arranged such that both electrodes extended the length of the chamber. Alternatively and preferably, both electrodes would be relatively short so as to extend only along a portion of the chamber. One electrode would be placed substantially closer to the start of the chamber than the other, without any overlap.

According to another preferred embodiment of the present invention, the device further includes a reference chamber for the determination of clotting in a control sample.

The principles and operation of a method and a device for the determination of clotting time according to the present invention may be better understood with reference to the drawings and the accompanying description, it being understood that these drawings are given for illustrative purposes only and are not meant to be limiting.

The following description is divided into three sections. The first section describes the measurement of capacitance in a liquid sample, such as a blood sample. The second section describes the measurement of resistance in the liquid sample. The third section describes the detection of the interaction of a ligand/receptor pair with the device of the present invention.

Section 1: Measurement of Capacitance

[Background]

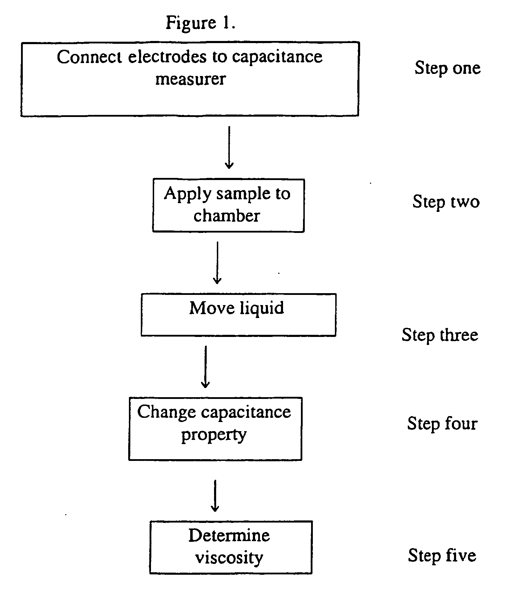

Referring now to the drawings, Figure 1 shows a flow chart of an exemplary method for determining the viscosity of a liquid. In step one, power is supplied to the electrodes. For example, a constant voltage could be applied across the electrodes. The electrodes are preferably flat, and are insulated from the liquid, such that the liquid does not directly contact the electrodes. The power being supplied should be alternating voltage.

In step two, a sample of the liquid is placed at the entrance to a chamber with two electrodes. The lower surface of the chamber could be any suitable shape including, but not limited to, a square, a rectangle, a circle and an ellipse. The chamber preferably features only a single capillary channel.

In step three, the liquid moves through the chamber through the action of a receiver, for example by capillary action, or by application of a vacuum, by gravity, or through mechanical or osmotic pressure, thereby increasing the surface area of the electrodes which is covered by the liquid, although the liquid does not directly contact any portion of the electrodes. Instead, the liquid spreads within the chamber, such that the volume of the liquid within the chamber is directly correlated to the measured capacitance. The vacuum could be applied by the presence of a suction device which is an example of the receiver. The capillary action could be caused by a capillary structure such as a capillary depth of the chamber, and/or a narrow channel shape of the chamber. The capillary depth of the chamber would be a depth selected to ensure that the liquid would enter the chamber through the capillary action.

In step four, the movement of the liquid causes an electrical property to alter as the volume occupied by the liquid increases. For example, the measured capacitance could increase. For the measurement of capacitance, a capacitance meter should be electrically connected to the electrodes. In step five, the alteration of the electrical property is used to determine the viscosity of the liquid sample.

The method of the present invention could be used to measure the viscosity of a blood sample as it clots, for example. The blood sample would be taken from the patient and then placed at the entrance to the chamber, as described previously. The clotting time could then be determined by measuring the capacitance after a fixed time period had elapsed, or by measuring the period of time required to reach the maximum capacitance, for example. In either case, the length of time required for the blood sample to clot would be directly related to the volume occupied by the sample before clotting occurred. For example, if the capacitance was measured after a fixed time period had elapsed, the amount of the various measured amounts of capacitance would increase as the clotting time also increased, as this would permit the blood sample to travel farther down the chamber before clotting occurred. Similarly, if the time period required to reach the maximum capacitance was measured, this time period would increase as the clotting time also increased. Thus, as the clotting time increased, the volume of the chamber occupied by the sample before clotting occurred would also increase.

The advantage of measuring the capacitance, rather than the amount of current passing between the two electrodes for example, is that the measurement of capacitance does not alter the sample itself. Since the sample substantially never directly contacts any portion of the electrodes, the properties of the sample are not altered by the electrodes and/or by the voltage supplied to the electrodes. By contrast, in order to measure current passing through the sample, at least a portion of the electrodes must directly contact the sample. Such direct electrical contact consumes electrochemical species in the blood sample, which in turn decreases the amount of current passing through the sample. However, movement of the blood sample farther into the chamber increases the volume of the chamber occupied by the sample, and hence increases the amount of current passing through the sample. These two opposing trends may decrease the accuracy of the measurement of blood coagulation. Thus, the measurement of capacitance is clearly more accurate.

Although the description of exemplary devices for measuring the viscosity of a liquid sample according to the present invention is specifically drawn to devices for determining the clotting time of a blood sample, it is understood that this is for the sake of clarity only and is not meant to be limiting. As noted previously, the method and device of the present invention could also be used to determine the viscosity of substantially any type of liquid sample, as such viscosity is directly related to the volume of the chamber occupied by the sample as the liquid sample moves through the chamber.

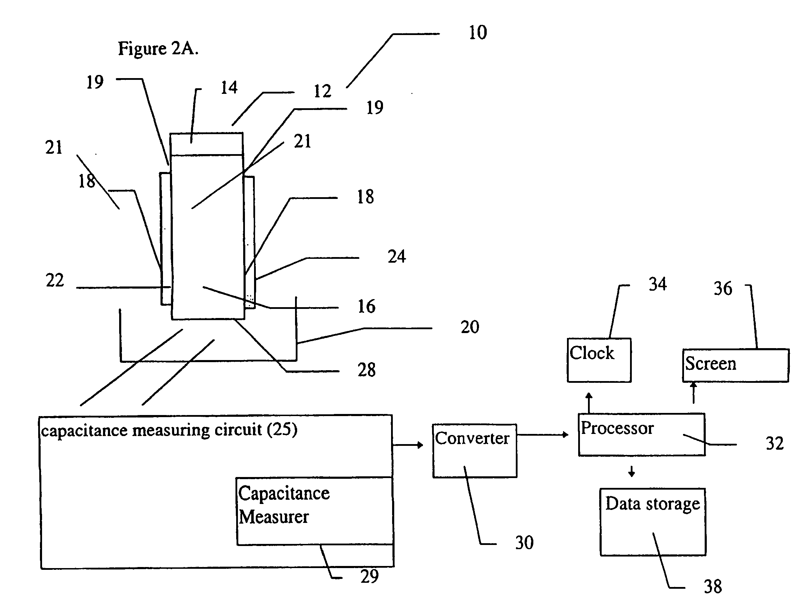

Figures 2A-2D are schematic block diagrams of exemplary devices for the determination of clotting time by measuring capacitance according to the present invention. Figure 2A shows a first embodiment of an exemplary device. A clotting diagnostic device **10** is shown with a sensor **12** inserted. Sensor **12** features a sample area **14** onto which a blood sample is placed (not shown). The blood sample is drawn into a test chamber **16** formed between two electrodes **18,** for example by capillary action. Alternatively and preferably, device **10** could include a pump for pumping the sample into test chamber **16,** or a suction device for sucking the sample into test chamber **16,** for example. The sample could also enter test chamber **16** through mechanical or osmotic pressure, or the application of a vacuum. Preferably, test chamber **16** is a single capillary channel as shown.

The sample is preferably not able to directly contact electrodes **18.** Electrodes **18** are preferably either insulated with an insulating layer (not shown) or are alternatively and preferably placed externally to walls **19** of test chamber **16,** both of which options are herein collectively described as an insulator **21.**

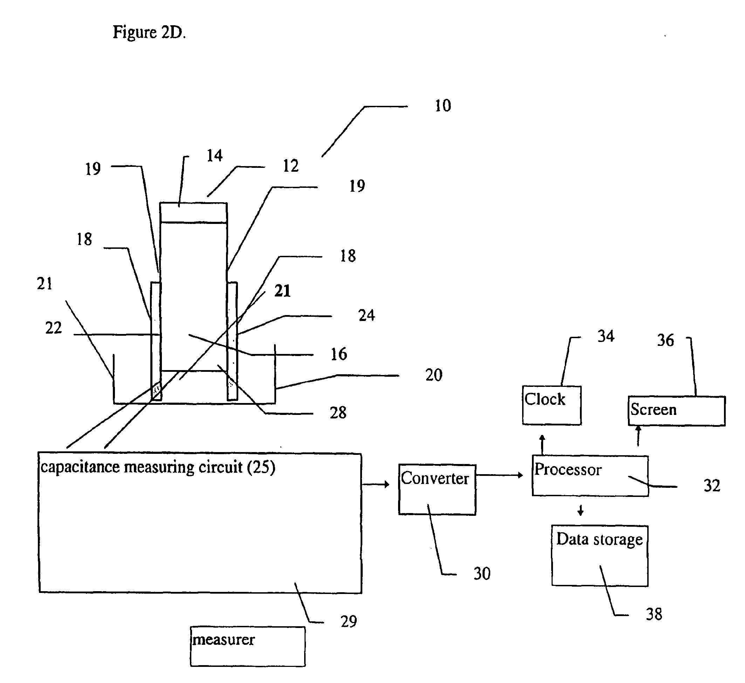

In this embodiment, both test chamber **16** and electrodes **18** are formed on sensor **12.** Alternatively and preferably, test chamber **16** and electrodes **18** could be formed within sensor receptacle **20** (not shown). Also alternatively and preferably, electrodes **18** could be formed within sensor receptacle **20,** but test chamber **16** could be formed on sensor **12,** as shown in Figure 2D. Since sensor **12** is preferably disposable for easy cleaning and maintenance of device **10,** the illustrated arrangement of either Figure 2A, in which both test chamber **16** and electrodes **18** are formed on sensor **12,** or of Figure 2D, in which electrodes **18** are formed within sensor receptacle **20,** and test chamber **16** is formed on sensor **12,** is preferred. For any of these embodiments, electrodes **18** are positioned such that the sample does not directly contact electrodes **18,** since electrodes **18** are insulated with insulator **21,** for example by being insulated with an insulating layer or by being placed outside walls **19.** Also for either embodiment, the blood sample is placed onto sensor **12** which is in contact with sensor receptacle **20.**

Sensor receptacle **20** is in electrical contact with a capacitance measurement circuit **25.** The design of capacitance measuring circuits, such as capacitance measuring circuit **25,** is well known in the art of electronic circuit design. Capacitance measuring circuits are incorporated in many commercially available multi-meter instruments, such as those manufactured by Thurlby Thandar Instruments (TTi 1705; Huntingdon, Cambridgeshire, UK), Fluke Technology (USA), Keithley Instruments (USA), and Appa Technology (APPA 93 Multimeter; Taiwan), or in dedicated capacitance meters, such as those manufactured by TES (TES-1500; Taiwan). Also shown is a measuring device for measuring capacitance, shown as a capacitance measurer **29.** As shown, capacitance measuring circuit **25** is attached to both electrodes **18,** designated as a first electrode 22 and a second electrode **24,** on sensor **12** Electrodes **18** are also shown disposed in parallel on opposing sides of test chamber **16,** since for proper and sensitive capacitance measurement, at least one electrode **18** should preferably be on each side.

Methods and materials for manufacturing electrodes **18** and sensor **12** are well known in the art. The material could be in the form of a metal foil or film, or carbon or metal impregnated film or metal sputtered film, for example. Sensor **12** could be a card made from any type of suitable plastic, preferably semi-rigid or rigid plastic. Electrodes **18** could be screen printed onto sensor **12** according to methods well known in the art. Chamber **16** could be formed onto sensor **12** according to various microfabrication techniques which are well known in the art (see for example U.S. Patent No. 5,120,420), such that electrodes **18** are insulated by an insulator **21.**

For example, sensor **12** could be a plastic card formed from polyester or polycarbonate. Silver paste could then be screen printed onto sensor **12** to form electrodes **18.** The assembly would then be dried. Insulating paste could optionally be screen printed onto sensor **12** such that both electrodes **18** are not exposed within chamber **16.** The assembly would again be dried. Finally, a spacer and cover made from plastic film or foil could be attached to sensor **12** in order to form a chamber **16** which could be a capillary channel.

In the embodiment shown, the blood sample is placed onto sample area **14** and is then drawn into test chamber **16** by capillary action. The dimensions of test chamber **16** should therefore permit such capillary action to occur. Such dimensions could be easily selected by one of ordinary skill in the art. An opposing end **28** of sensor **12,** opposing sample area **14,** is then placed in contact with sensor receptacle **20,** for example by inserting sensor **12** into sensor receptacle **20.**

Alternatively and more preferably, opposing end **28** of sensor **12** is first placed in contact with sensor receptacle **20.** Next, voltage, preferably alternating, is applied to electrodes **18** from capacitance measurement circuit **25.** Finally, the blood sample is placed onto sample area **14** by the patient, for example by using a needle (not shown) to break the skin of the patient and to allow the blood sample to be collected. The term "needle" includes any sharp point or edge which can break the skin of the patient. In this embodiment, device **10** could be a kit for determining the clotting time of the blood sample from the patient in the home testing environment.

This particular mode of operation is more preferred because the application of the blood sample can automatically trigger the timing function. For example, when sensor **12** is placed in contact with sensor receptacle **20** before a blood sample has been applied, the measured capacitance would be low. As soon as the blood sample was applied, the measured capacitance would increase, enabling device **10** to automatically detect the presence of the blood sample.

As blood sample flows through test chamber **16,** the liquid flows between the electrodes **18,** one on either side of test chamber **16,** causing an increase of, the electrical capacitance between first electrode **22** and second electrode **24.**

After measuring the capacitance, meter **29** then converts the measured capacitance to a digital signal by a converter **30.** The digital signal is then sent to a processor **32** for analysis of the signal and the calculation of the clotting time from the signal. Processor **32** includes instructions for calculating the clotting time from the signal. Processor **32** also includes a clock **34** for accurately measuring time. Such clocks suitable for micro-processors and other computational devices are well known in the art. Processor **32** could be a dedicated microprocessor with ROM (read-only memory) memory for storing the instructions, for example. Circuits which perform calculations for performing a diagnostic test according to the electrical property of a sample of blood already exist in portable electrochemical blood glucose meters, such as those distributed by Bayer, under the trade name of Glucometer Elite™, and by Medisense, under the trade name Exactech™.

Preferably, the calculated clotting time is displayed on a display screen **36.** Also preferably, the clotting time, and any other desired information, is stored in a data storage area **38,** which could be a flash memory array for example.

The clotting time could be calculated in a variety of ways. For example, the blood sample could be allowed to flow through test chamber **16** until clotting had occurred, at which point blood sample would cease to flow. The cessation of blood flow through test chamber **16** could be determined by noting the time point at which the amount of capacitance reaches a maximal value. The clotting time would then be determined according to this time point. Alternatively and preferably, the amount of capacitance could be measured at a fixed time point, after a predetermined period of time had elapsed from the time at which sensor **12** was placed in contact with sensor receptacle **20.**

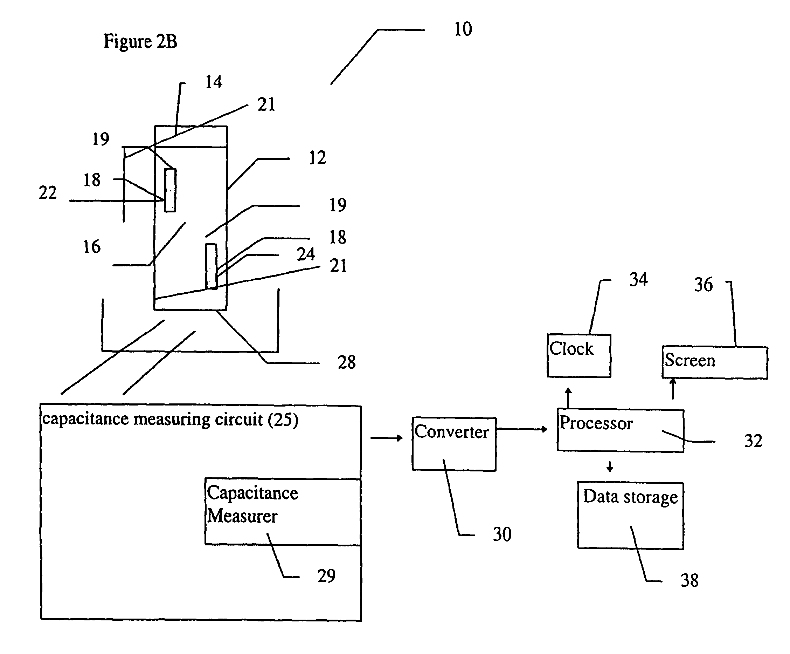

According to yet another preferred embodiment of the device of the present invention, shown in Figure 2B, electrodes **18** could be arranged such that neither electrode **18** extends along substantially a majority of, or substantially the entirety of, the length of test chamber **16.** Instead, preferably, both electrodes **18** are relatively short so as to extend only along a portion of walls **19** of test chamber **16,** this portion being substantially shorter than the majority of the length of test chamber **16.** First electrode **22** would be placed substantially near the start of test chamber **16,** close to sample area **14.** Second electrode **24** would then be placed farther along test chamber **16,** substantially closer to opposing end **28** of sensor **12.** Of course, the positions of first electrode **22** and second electrode **24** could also be reversed (not shown). In any case, first electrode **22** and second electrode **24** should not be placed such that there is any overlap between first electrode **22** and second electrode **24.** As for Figure 2A, electrodes **18** are also shown disposed in parallel on opposing sides of test chamber **16.** Again, optionally and preferably, both electrodes **18** could be placed on one side of walls **19** of test chamber **16** in series, one after the other (not shown).

In this embodiment, the time required to substantially increase the capacitance is determined in order to calculate the clotting time, rather than measuring the amount of capacitance. The time required to increase the capacitance to a predetermined level is the period of time required for the blood sample to proceed from first electrode **22,** which is closer to sample area **14,** to second electrode **24,** such that the blood sample is in close proximity both to first electrode **22** and to second electrode **24.** Alternatively and preferably, the amount of time during which the capacitance increases could also be measured, such that the time would be measured until the capacitance reaches a plateau and stops increasing. These embodiments have an advantage since the actual value of the capacitance is not important for the measurement of the clotting time, but only relative changes in the capacitance.

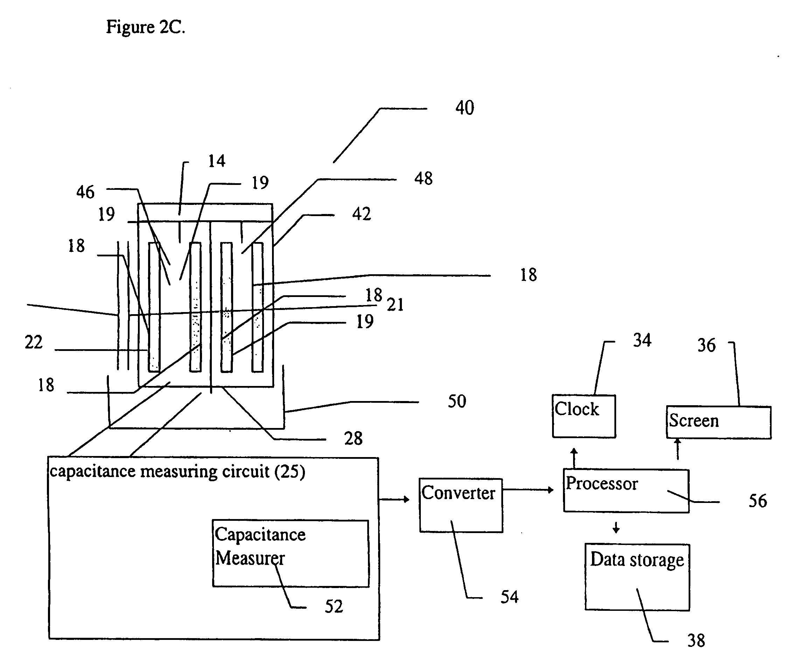

Figure 2C is a block diagram of a third exemplary embodiment, in which the device is able to compare the clotting time of the blood sample with the behavior of a control sample. In this embodiment, a clotting diagnostic device **40** also features a sensor **42.** Sensor **42** also has sample area **14.** Blood is placed on sample area **14** and then flows into two chambers **44,** a test chamber **46** and a reference chamber **48.** Preferably, the blood sample is divided equally, such that a substantially equal portion of the sample flows into each channel **44.** Both test chamber **46** and reference chamber **48** each feature two electrodes **18,** as described previously. Preferably, both chambers **46** and **48** can share one reference or ground electrode (the center one), thereby simplifying electrical circuits and saving space on the sensor. Although electrodes **18** are shown arranged as for the first embodiment of Figure 2A, electrodes **18** could also be arranged according to the second embodiment shown in Figure 2B. As for both Figures 2A and 2B, electrodes **18** are also shown disposed in parallel on opposing sides of test chamber **46** or of reference chamber **48.** Again, both electrodes **18** could be placed on one side of test chamber **46** or of reference chamber **48** (not shown). The surface of electrodes **18** is preferably insulated by an insulator **21,** for example by being placed outside wall or walls **19,** or by being insulated with an insulating layer (not shown), and does not contact the tested liquid. Furthermore, test chamber **46** is functionally equivalent to chamber **16,** as described for Figures 2A and 2B.

Reference chamber **48** preferably features at least one factor for altering the clotting behavior of the blood sample, present either as a liquid droplet for a wet reagent, or dried for a dry reagent. For example, reference chamber **48** could include a predetermined amount of an anti-coagulant factor, such that the clotting time of the portion of the blood sample in reference chamber **48** is increased. Alternatively, reference chamber **48** could include a predetermined amount of a factor for inducing coagulation, such that the clotting time of the portion of the blood sample in reference chamber **48** is decreased. More preferably, test chamber **46** also contains such a reagent, either wet or dried. Chamber **16** of Figures 2A and 2B could also contain such a reagent. In any case, the selection of the reagent for test chamber **46,** reference chamber **48** or both would depend upon the type of test being performed and could easily be selected by one of ordinary skill in the art.

Sensor **42** is placed in contact with a receptacle **50** to perform the measurement of clotting time, as described for Figure 2A. Receptacle **50** is also in electrical contact with capacitance measuring device 52, and is able to measure the capacitance between both sets of electrodes **18** from test chamber 46 and reference chamber **48.** Similarly, a converter **54** should be able to separately convert each capacitance measurement to a digital signal. Both the test digital signal from test chamber **46,** and the reference digital signal from reference signal **48,** are then used by a processor **56** to calculate the clotting time.

The altered clotting behavior of the blood sample in reference chamber **48** is used to adjust the apparent clotting time of the blood sample as determined through test chamber **46.** Such an adjustment acts as a control for any individual characteristics of the blood sample. These variations from the expected behavior of the blood sample can be compensated by the comparison of the amount of capacitance or the clotting time of the blood in reference chamber **48** and test chamber **46**. Thus, the third preferred embodiment of the present invention as shown by device **40** is able to compensate for deviations from the theoretical clotting time of the blood sample in order to more accurately determine the true clotting time, or other coagulation characteristic, of the blood sample.

Figure 2D shows yet another embodiment, which is substantially similar to the embodiment shown in Figure 2A, except that electrodes **18** are formed within sensor receptacle **20,** while test chamber **16** is formed on sensor **12.**

As noted previously, both embodiments of the device of the present invention are based upon the determination of the movement of the blood sample through the test chamber and, if present, the reference chamber. Such a determination is performed by measuring the capacitance between the electrodes disposed on opposing sides or on the same side of the chamber, but insulated from the test liquid or simply located externally to the chamber. As the blood advances through the chamber, the volume of liquid in the chamber (and between the electrodes) will increase proportionally and will, likewise, cause a proportional increase in the electrical capacitance between the electrodes, as demonstrated for example in U.S. Patent No. 5,720,733.

For any of these embodiments, the chamber can be pretreated before the sample is analyzed, for example by soaking in a buffer containing a detergent and/or a hydrophilic polymer. Examples of such hydrophilic polymers include but are not limited to various kinds of polysaccharides, PVA (Poly Vinyl Alcohol), PVP (PolyVinyl Pyrrolidone), and proteins.

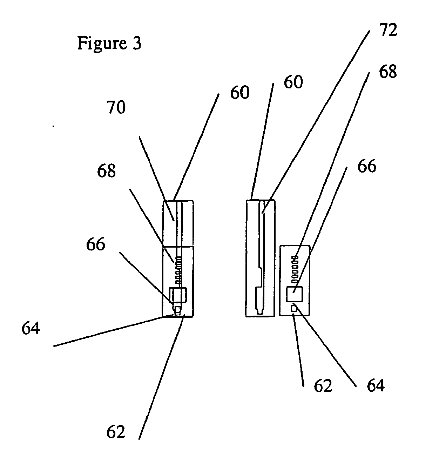

Figure 3 is a schematic block diagram of yet another embodiment of the device of the present invention, featuring apertures in the cover of the sensor. These apertures are preferably placed in the cover of both the test and the reference chamber when both are present, or else simply in the cover of the test chamber.

According to one preferred design, a sensor **60** has a cover **62,** sensor **60** being shown with cover **62** in place (left) and without cover **62** in place (right). As shown, cover **62** preferably features at least one aperture. More preferably, cover **62** features a plurality of apertures. A first aperture **64** is located over the area for inserting the sample when cover **62** is in place on top of sensor **60.** Another aperture **66** and a plurality of smaller apertures **68** are dispersed along the length of a test/reference chamber **70** formed on sensor **60.**

Apertures such as apertures **64, 66** or **68,** alone or in combination, improve the degree of control exertable over the speed of advancement of the sample. In addition, when a reagent is present in the chamber, the apertures improve the extent of mixing of the sample with the reagent when such a reagent is present. As a result, the precision of the measurement is greatly improved. Indeed, increasing the extent to which the test chamber is uncovered slows the progression of the fluid of the sample, thereby improving the precision and the repeatability of the measurements. By slowing the progression of the fluid through the test chamber, a smaller and/or shorter device can be manufactured, which is in turn easier to handle, store and transport.

Section 2: Measurement of Resistance

Although the viscosity of the liquid sample, and in particular the clotting time of a blood sample, can be measured through capacitance as described previously, these properties of the sample can also be measured through resistance. This alternative embodiment of the present invention, which could optionally be incorporated in a single device with the previously described embodiment of the present invention, is described in greater detail below.

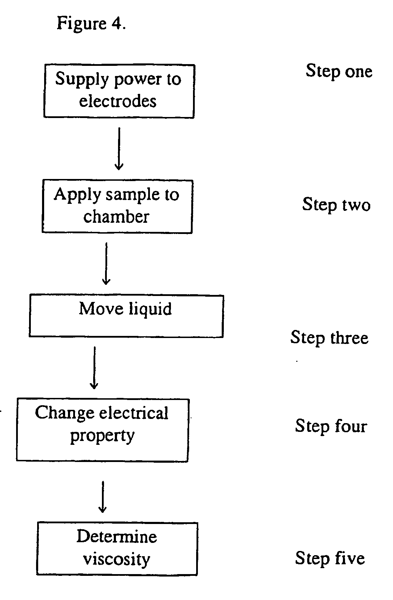

Referring now to the drawings, Figure 4 shows a flow chart of an exemplary method for determining the viscosity of a liquid through the measurement of resistance according to the present invention. In step one, power is supplied to the electrodes. For example, a constant voltage could be applied across the electrodes. In step two, a sample of the liquid is placed at the entrance to a chamber with two electrodes. The lower surface of the chamber could be any suitable shape including, but not limited to, a square, a rectangle, a circle and an ellipse. Preferably, the chamber would feature a single capillary channel.

In step three, the liquid moves through the chamber through the action of a receiver, for example by capillary action, or by application of a vacuum, by gravity, or through mechanical or osmotic pressure, thereby increasing the surface area of the electrodes which is covered by the liquid. The vacuum could be applied by the presence of a suction device which is an example of the receiver. The capillary action could be caused by a capillary structure such as a capillary depth of the chamber, and/or a narrow channel shape of the chamber. The capillary depth of the chamber would be a depth selected to ensure that the liquid would enter the chamber through the capillary action.

In step four, the movement of the liquid causes an electrical property to alter as the covered surface area of the electrodes increases. For example, the measured current could increase, or the resistance could decrease. In step five, the alteration of the electrical property is used to determine the viscosity of the liquid sample.

This embodiment of the method of the present invention could be used to measure the viscosity of a blood sample as it clots, for example. The blood sample would be taken from the patient and then placed at the entrance to the chamber, as described previously. The clotting time could then be determined by measuring the current after a fixed time period had elapsed, or by measuring the period of time required to reach the maximum current, for example. In either case, the length of time required for the blood sample to clot would be directly related to the surface area of the electrodes covered by the sample before clotting occurred. For example, if the current was measured after a fixed time period had elapsed, the amount of current would increase as the clotting time also increased, as this would permit the blood sample to travel farther down the chamber before clotting occurred. Similarly, if the time period required to reach the maximum current was measured, this time period would increase as the clotting time also increased. Thus, as the clotting time increased, the surface area of the electrodes covered by the sample before clotting occurred would also increase.

Although the description of exemplary devices for measuring the viscosity of a liquid sample according to the present invention is specifically drawn to devices for determining the clotting time of a blood sample, it is understood that this is for the sake of clarity only and is not meant to be limiting. As noted previously, the method and device of the present invention could also be used to determine the viscosity of substantially any type of liquid sample, as such viscosity is directly related to the surface area of the electrodes covered by the sample as the liquid sample moves through the chamber.

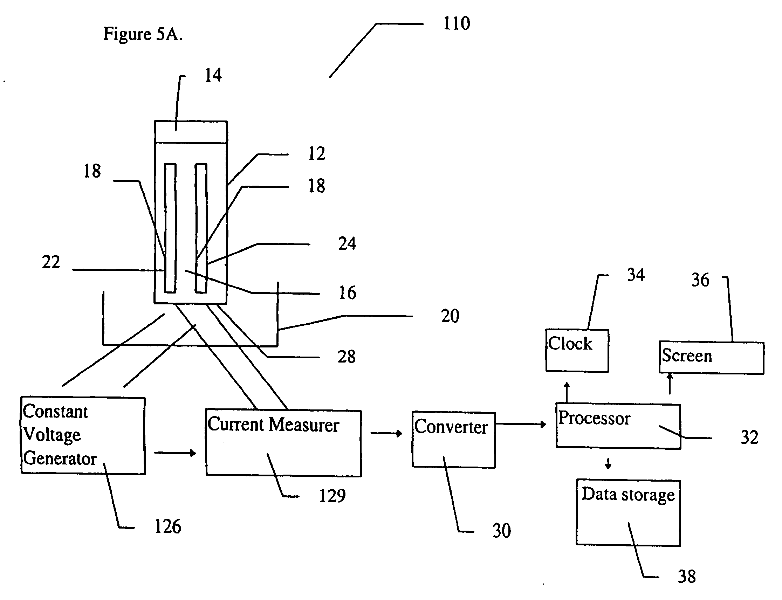

Figures 5A-5C are schematic block diagrams of exemplary devices for the determination of clotting time by resistance according to the present invention, which are similar in certain respects to Figures 2A-2D described previously. Those features which are identical to the devices of Figures 2A-2D are labeled with the same reference numbers in Figures 5A-5C.

Figure 5A shows a first embodiment of an exemplary device for determining the viscosity of a liquid, such as a blood sample, by the measurement of resistance. A clotting diagnostic device **110** is shown, which differs from clotting device **10** of Figure 2A in several respects. First, the power source which is in electrical contact with sensor receptacle **20** is preferably a constant voltage generator **126.** Constant voltage generator **126** is attached to both electrodes **18.** As shown, first electrode **22** is the positive electrode and second electrode **24** is the negative electrode, but polarity may be reversed without affecting the functionality of the device.

Second, preferably opposing end **28** of sensor **12** is first placed in contact with sensor receptacle **20** and then voltage is applied to electrodes **18** from constant voltage generator **126.** In this embodiment, device **110** could be a kit for determining the clotting time of the blood sample from the patient in the home testing environment.

This particular mode of operation is more preferred because the application of the blood sample can automatically trigger the timing function. For example, when sensor **12** is placed in contact with sensor receptacle **20** before a blood sample has been applied, the measured current would be approximately zero. As soon as the blood sample was applied, the measured current would immediately and rapidly increase, enabling device **110** to automatically detect the presence of the blood sample.

As blood sample flows through test chamber **16,** the liquid contacts both electrodes **18,** one on either side of test chamber **16.** Such contact enables electrical current to move from first electrode **22** to second electrode **24.** As more liquid flows into test chamber **16,** an increasing amount of electricity flows from first electrode **22** to second electrode **24,** thus increasing the amplitude of the current.

As electrical current flows from first electrode **22** to second electrode **24,** the amplitude of the current is measured by an exemplary current measuring device **129,** which is the second important difference from device **10** of Figure 2A. Since the current is measured at a fixed voltage, the changes in resistance as liquid flows over first electrode **22** and second electrode **24** could also be measured, provided the applied voltage is sufficiently high to result in an easily detectable current. Indeed, glucose meters for the home testing environment also measure current. Thus, the measurement of current is preferred in a device for the home testing environment since the electronic equipment required is more suitably robust.

After measuring the current, current measuring device **129** then converts the measured current to voltage. The analog voltage is then converted to a digital signal by converter **30,** and the digital signal is then sent to a processor **32** for analysis of the signal and the calculation of the clotting time from the signal. Processor 32 again includes instructions for calculating the clotting time from the signal. Again, processor **32** also includes a clock **34** for accurately measuring time. Such clocks suitable for micro-processors and other computational devices are well known in the art. Processor **32** could be a dedicated microprocessor with ROM (read-only memory) memory for storing the instructions, for example. Such current measuring circuits already exist in portable electrochemical blood glucose meters, such as those distributed by Bayer, under the trade name of Glucometer Elite™, and by Medisense, under the trade name Exactech™.

The clotting time could be calculated in a variety of ways. For example, the blood sample could be allowed to flow through test chamber **16** until clotting had occurred, at which point blood sample would cease to flow. The cessation of blood flow through test chamber **16** could be determined by noting the time point at which the amplitude of the current reaches a maximal value. The clotting time would then be determined according to this time point. Alternatively and preferably, the amplitude of the current could be measured at a fixed time point, after a predetermined period of time had elapsed from the time at which sensor **12** was placed in contact with sensor receptacle **20.**

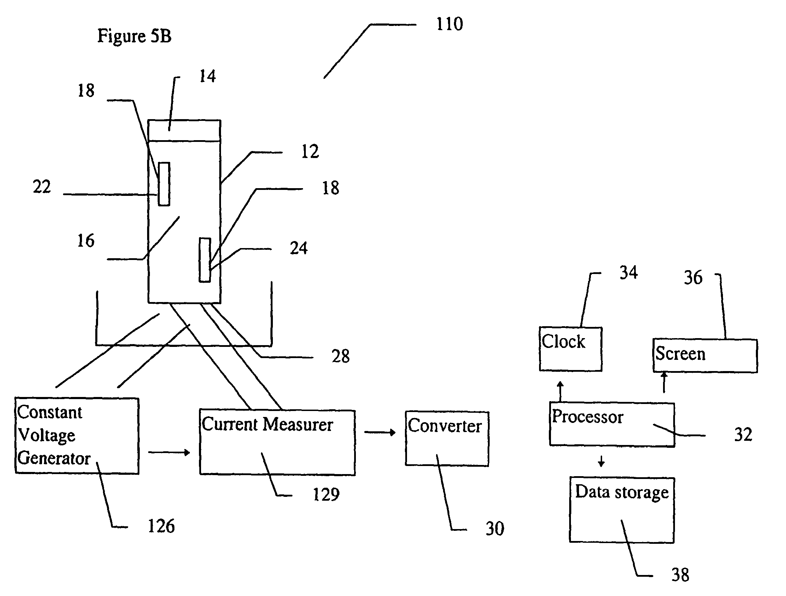

According to yet another preferred embodiment of the device of the present invention for measuring the viscosity of a liquid through the determination of resistance, shown in Figure 5B, electrodes **18** could be arranged such that neither electrode **18** extends along substantially a majority of, or substantially the entirety of, the length of test chamber **16.** Instead, preferably, both electrodes **18** are relatively short so as to extend only along a portion of test chamber **16,** this portion being substantially shorter than the majority of the length of test chamber **16,** in an arrangement which is similar to that shown in Figure 2B.

In this preferred embodiment, the time required to close the circuit is determined in order to calculate the clotting time, rather than the amount of current. The time required to close the circuit is the period of time required for the blood sample to proceed from first electrode **22,** which is closer to sample area **14,** to second electrode **24.** This embodiment has the advantage of simplified electrical measurement since the measurement of amplitude is not important, while the time period required for closing the circuit is easily and inherently determined by any microprocessor or a timer circuit which are well known in the art.

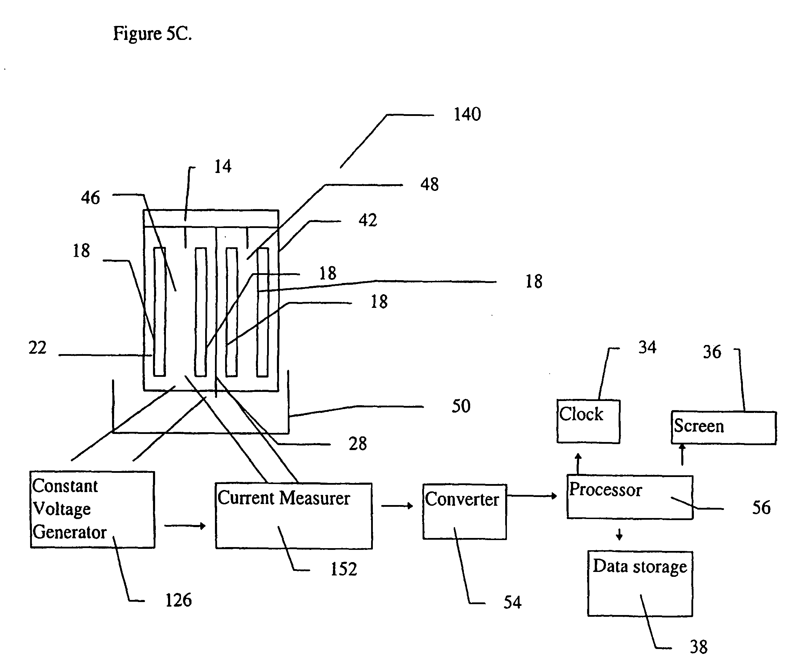

Figure 5C is a block diagram of a third exemplary embodiment, in which the device of the present invention is able to compare the clotting time of the blood sample with the behavior of a control sample. In this embodiment, a clotting diagnostic device **140** is similar to clotting diagnostic device **40** of Figure 2C. However, receptacle **50** is now in electrical contact with constant voltage generator **126.** A current measuring device **152** is able to receive current from both sets of electrodes **18** from test chamber **46** and reference chamber **48** and to convert the measured current to voltage. Similarly, an analog to digital converter **54** should be able to separately convert each analog signal to a digital signal. Both the test digital signal from test chamber **46,** and the reference digital signal from reference signal **48,** are then used by processor **56** to calculate the clotting time.

The altered clotting behavior of the blood sample in reference chamber **48** is used to adjust the apparent clotting time of the blood sample as determined through test chamber **46.** Such an adjustment acts as a control for any individual characteristics of the blood sample. These variations from the expected behavior of the blood sample can be compensated by the comparison of the amplitude of the current or the clotting time of the blood in reference chamber **48** and test chamber **46.** Thus, the third preferred embodiment of the present invention as shown by device **140** is able to compensate for deviations from the theoretical clotting time of the blood sample in order to more accurately determine the true clotting time, or other coagulation characteristic, of the blood sample.

As noted previously, this embodiment of the device of the present invention are based upon the determination of the movement of the blood sample through the test chamber and, if present, the reference chamber. Such a determination is performed in this embodiment by measuring the electrical current passing through the electrodes disposed on opposing sides or on the same side of the chamber . As the blood advances through the chamber , the surface of the electrodes covered by the blood will increase proportionally. If a constant voltage is applied to the electrodes, the electrical current will be proportional to the resistance of the system, which includes the components of the device and the blood sample itself. Such proportionality can be demonstrated by the following equations:

$$V=iR$$

where V is voltage, R is resistance and i is current. This equation can be rearranged to:

$$i=V/R$$

However, resistance can be determined as follows:

$$R=\rho \, L/A$$

where p is the specific resistance of the material, L is the distance between the two electrodes and A is the area of the electrodes. Therefore,

$$i=VA/(\rho L)$$

such that p and L are constant. If voltage is then held constant, current (i) is proportional to the surface area (A) of the electrodes covered by the blood sample and hence to the clotting time of the blood sample.

<u>Example 1</u>

<u>Construction and Testing of a Clotting Time Device</u>

The device, including the sensor, was constructed according to the first example of this embodiment of the device of the present invention, as shown in Figure 5A. The sensor was constructed from a flat plastic base card, by building an approximately 1.5 mm wide and approximately 20 mm long capillary channel over the two conductive carbon tracks. The sides of the channel were built from double sided adhesive tape, about 200 microns deep. The channel was covered with a section of transparent polyester film. The arrangement was designed to permit a reference portion of a five microliter blood sample to travel along the entire length of the capillary channel, while a second portion of the same sample remains at the sample origin, thus preventing limitation of measurement by the sample size.

The electrodes were then attached to a standard voltage generating device, and the resulting current was then measured by a standard device for measuring current or voltage. Both of these devices are well known to those of ordinary skill in the art. It should be noted that this sensor was intended as an example for experimental testing only and was not meant to be limiting in any way.

For the specific tests described below, the volume of the blood sample was five microliters and a 1.5 V voltage was applied from the voltage generating device.

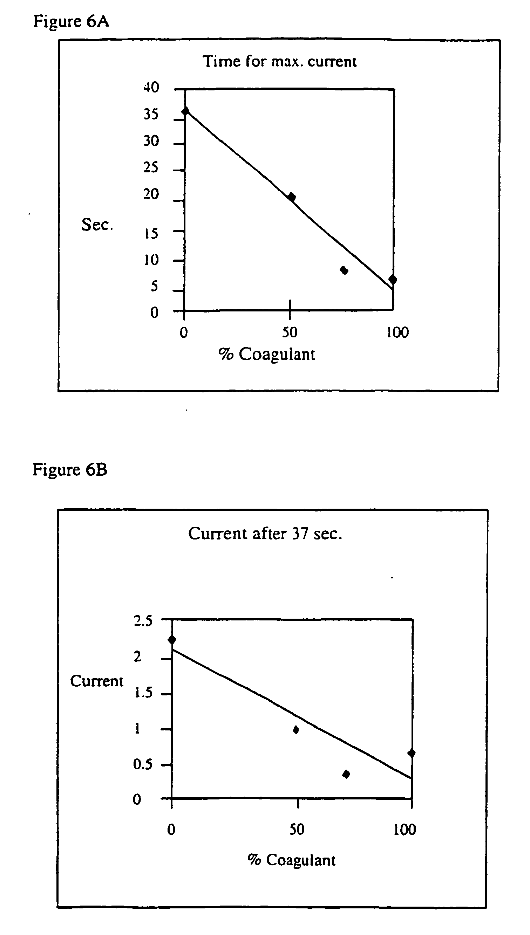

Variation between blood samples was simulated by generating a series of blood samples to which varying amounts of coagulants were added. Figures 6A-6C show the effect of the addition of coagulant to blood samples on the measurement of current by the device. Figure 6A shows the decrease in the amplitude of the current (micro-amperes) as the percentage of coagulant added to the sample is increased. The amplitude of the current was measured 37 seconds after the sample was placed in the sensor. Figure 6B shows the decrease in the elapsed time (shown in seconds) required for the maximum current to be reached as the percentage of coagulant added to the sample is increased. Clearly, both the current at 37 seconds and the elapsed time required for the maximum current is related to the amount of coagulant added to the sample.

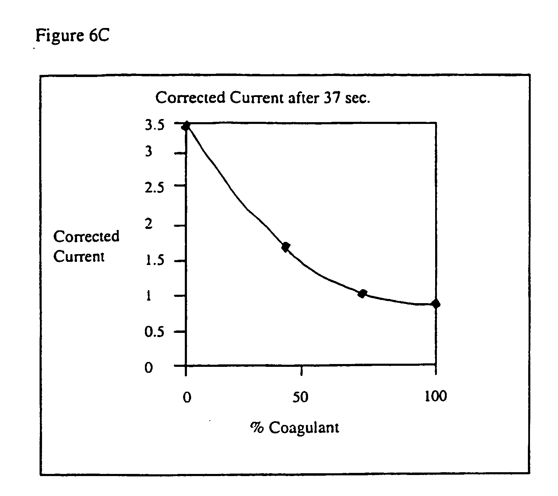

Figure 6C shows the corrected measured current from Figure 6A. The correction was performed as follows. The current (I) was measured at time zero ($I_0$), as soon as the blood sample was placed on the sensor (the sensor was already attached to the remainder of the device). The current was then measured again after one second had elapsed ($I_1$). The time period of one second was chosen so as to measure the current after a minimum time period had elapsed and before coagulation had started. Next, the average of $I_0$ and $I_1$ was determined ($I_a$). Finally, for each point of the transient curve of Figure 6A ($I_i$), the correction was performed as follows:

$$I_{corrected} = ( I_i - I_a )/ I_a$$

Such a method is an example of a method for correcting the measured current without using a reference chamber.

<u>Example 2</u>

<u>Construction and Testing of a Clotting Time Device with Dried Reagents</u>

An exemplary device of the present invention was also constructed according to the first embodiment of the device of the present invention, as shown in Figure 5A. In this case, however, coagulation reagents (Sigma Chemical Co) were dried in one area of the sensor. It should be noted that this device was also intended as an example for experimental testing only and was not meant to be limiting in any way.

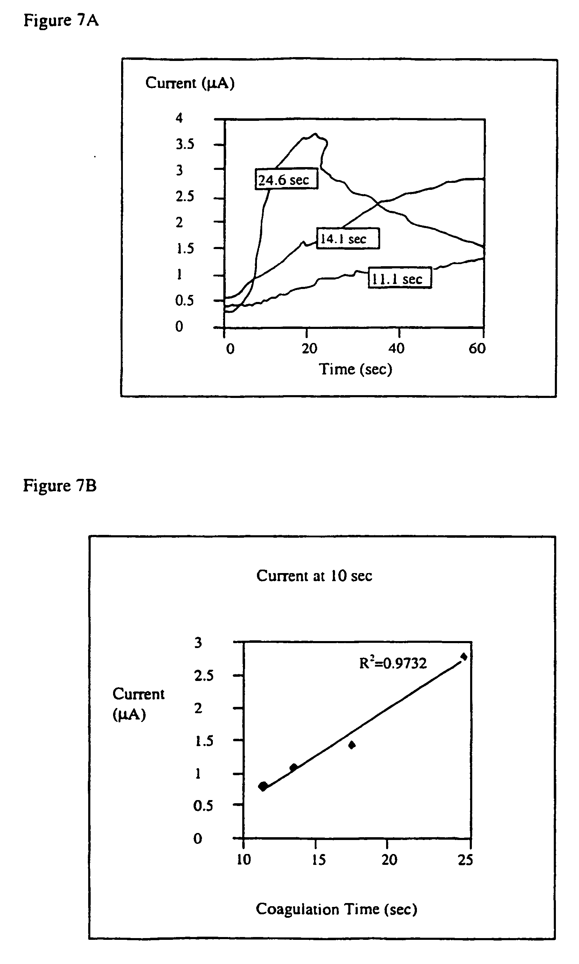

Figure 7A shows the variation of the current (micro-amperes) over time (seconds) for three different blood samples from patients undergoing anticoagulant therapy. These samples had different clotting times, which were measured as prothrombin time. Prothrombin time was determined both by the sensor, as shown in the graph, and with a standard reference method, employing an ACL1000 instrument. The clotting times obtained with the standard instrument are shown inside rectangles, attached to each of the current transient curves of the respective samples.

The measurement of prothrombin time with the ACL1000 instrument involved the separation of the plasma from the whole blood. Next, the plasma was mixed with a coagulant reagent mixture, which includes tissue factors and calcium ions. An equivalent coagulant reagent mixture was also dried onto the sample area of the sensor of the present invention. The clotting time was then determined by measuring the change in optical density of the sample with the ACL1000 instrument.

With regard to the curves determined with the device according to the present invention, clearly, the time required to reach the maximum measured current, which occurs at the time of clotting of the blood, was different for blood samples with different clotting times as determined by the ACL1000 instrument.

Figure 7B shows the quantitative relationship between the measured current 10 seconds after the blood sample was applied to the sensor inserted in the device of the present invention, and the coagulation time as determined by the standard reference method. Clearly, there is a directly proportional relationship between the current measured 10 seconds after application of the sample and the coagulation time, as determined by the reference method.

<u>Example 3</u>

<u>Construction and Testing of a Clotting Time Device</u>

<u>with Dried Reagents</u>

An exemplary device of the present invention was also constructed according to the first embodiment of the device of the present invention, as shown in Figure 5A. However, as further detailed below, certain modifications were made to the device. In particular, the carbon electrodes were subjected to a pretreatment, and a particular formulation of coagulation reagents was dried onto the sample area of the sensor of the present invention. It should be noted that this device was also intended as an example for experimental testing only and was not meant to be limiting in any way.

The preparation of this exemplary device according to the present invention was as follows. First, the carbon electrodes were cut and then pretreated by soaking in a buffer containing 0.1 M $Na_2CO_3$, 10 mg/ml BSA and 0.1% Tween-20 for 10 minutes at room temperature. Next, the electrodes were washed four times by dipping in distilled water. The electrodes were then dried over a hot plate at 75 °C.

The coagulation reagents were then prepared from lyophilized recombinant human thromboplastin reagent Innovin® (Dade International, USA). The active ingredients of Innovin® are a mixture of dried recombinant human tissue factor, which starts the coagulation cascade, and calcium ions. These active ingredients promote coagulation of the blood sample.

The contents of a 10 ml vial of Innovin® was resuspended with 2.5 ml of distilled water. This was supplemented with 200 mg/ml Mannitol (BDH, United Kingdom) and 0.1% Bioterge AS 40 (Stepan Europe, France).

The sensor of the present invention was then constructed with a modified spacer assembly as follows. First, the spacer was created by cutting a piece of 210 micron double coated polyester film (MS1182, Duramark, USA) to the desired shape. Squares of paper (5 mm by 7 mm) were then cut from Kimwipes® EX-L (Kimberly-Clark, USA) and were attached to the spacer at the sample area, such that substantially the entirety of the sample area up to the beginning of the capillary channels was covered with paper, to form the spacer assembly.

The spacer assembly was then attached to the electrodes. Eight microliters of reagent were applied to the paper lining the sample area of the spacer assembly and dried overnight at 37 °C in a turbo oven (Series 9000, Scientific). After drying, a piece of a transparency film (9 mm by 48 mm) was attached to the remaining free adhesive area of the spacer assembly, thus creating the capillary channels through which the blood sample flows.

The determination of clotting time by using this exemplary device was performed by inserting the sensor into the apparatus for measuring current. The power supply was set to supply 1500 mV of electricity to the electrodes. Next, a blood sample of 30 microliters was added to the paper at the sample area of the sensor, where the sample mixed with the dried reagent. The addition of the paper to the sample area had the advantage of promoting better mixing of the blood sample with the dried reagent material. Paper is an example of a substantially porous material which preferably at least partially covers the sample area in order to promote mixing of the blood and dried reagents. As for previous embodiments, the blood sample then flowed from the sample area through the capillary channels.

As soon as the blood sample contacted the electrodes, the apparatus began the measurement of elapsed time. Substantially simultaneously, the supply of electricity from the power source to the electrodes was temporarily suspended for about 90 seconds, until the blood sample ceased flowing forward through the capillary channel. At that moment, electricity was again supplied to the electrodes for about 4 seconds and the current was recorded.

Table 1 below shows the current measured 4 seconds after the electricity supply to the electrodes was re-established. The International Normalized Ratio is an adjusted value of clotting time, which is calculated as $R^{ISI}$, wherein R is the ratio of the prothrombin time determined for the blood sample and the mean of prothrombin times for normal subjects, and *ISI* is the International Sensitivity Index, which is a measure of the potency of the thromboplastin reagent employed in the assay. The value of *ISI* for Innovin® was provided by the manufacturer.

Table 1. Current Measured after Clotting

| Sample Number | International Normalized Ratio | Current (micro-amperes) |
|---|---|---|
| one | 0.98 | 0.281 ± 0.28 |
| two | 3.48 | 3.821 ± 0.071 |

As shown from these two samples, clearly the amount of current measured after clotting has occurred is directly related to the time of clotting. The device of this Example has the advantage of potentially greater reproducibility of results, as well as the ability to provide accurate results regardless of variations in the surface of the plastic substrate and the material of the electrodes.

Example 4

Determination of Clotting Time with a Sensor Strip

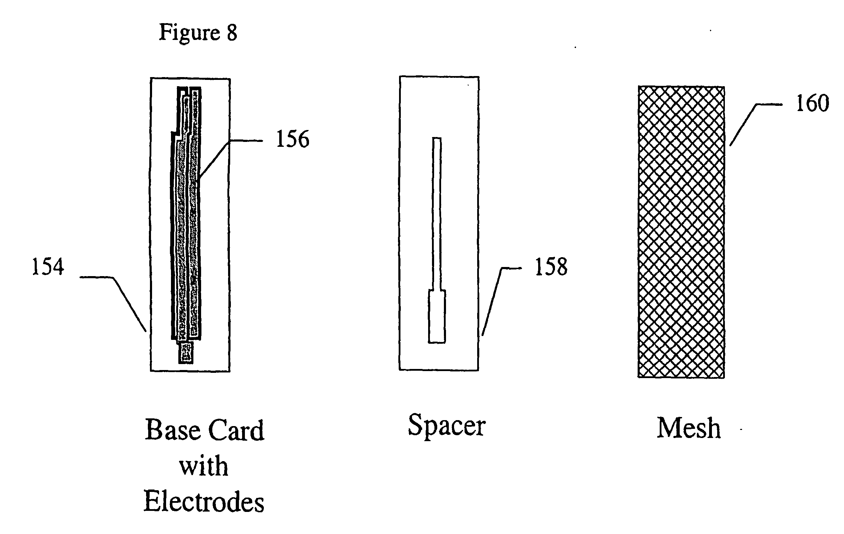

An exemplary device of the present invention was also constructed according to the first embodiment of the device of the present invention, as shown in Figure 5A. However, as further detailed below, certain modifications were made to the device. In particular, a nylon mesh was placed over the spacers in order to create the capillary channel, as shown in Figure 8. It should be noted that this embodiment could be altered to include the cover with apertures of Figure 3. Similarly, the cover with apertures of Figure 3 could be replaced by the mesh shown in Figure 8. The device was prepared as follows.

1. First the sensor strip was prepared. Clotting time sensor strips were constructed from the following parts, which are depicted in Figure 8. A Lexan® polycarbonate base card **154** featured two printed carbon electrode tracks **156.** For the purposes of this example, the dimensions of base card **154** were approximately 60 mm x 5 mm, although of course these dimensions could be altered. A spacer **158** was die-cut from a 2-mil thick polyester sheet, double coated with an acrylic pressure sensitive adhesive (Adhesives Research, type7810). Spacer **158** was attached to base card **154** so that the open slit in spacer **158** was centered over the space between electrodes **156,** thereby exposing a part of both electrodes **156.** The width of the slit was 1 mm and its length was 30 mm. The reagent/ specimen area had a width of 3 mm and a length of 10 mm. Next, a nylon mesh 160 (Nybolt 95T from Sefar, Ruschlikon, Switzerland) was attached to the top of spacer **158,** thus creating a capillary channel.

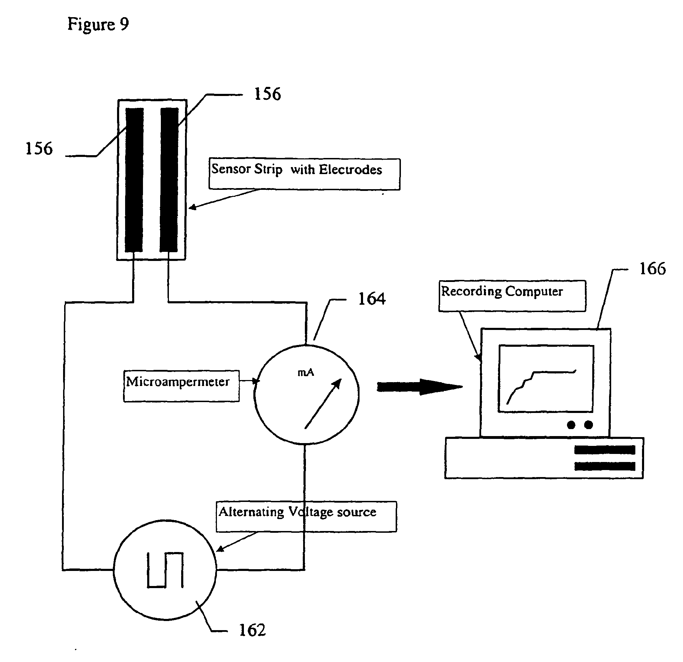

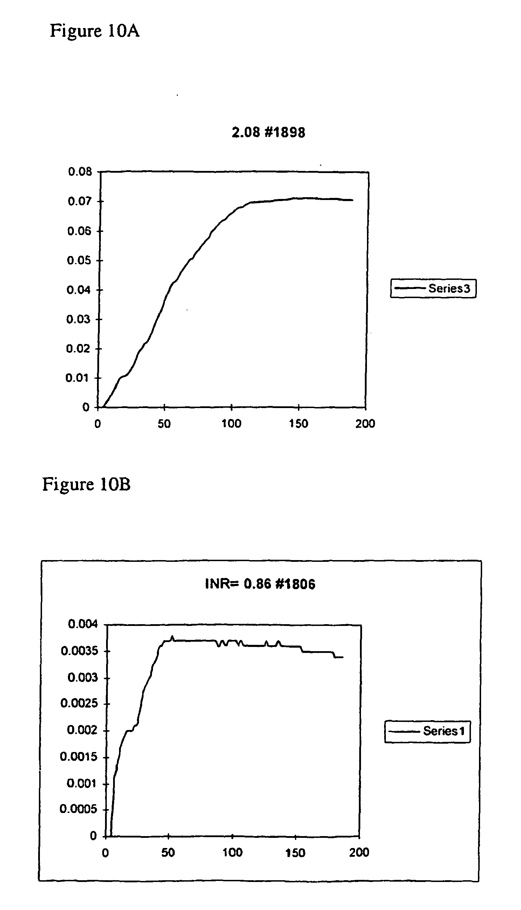

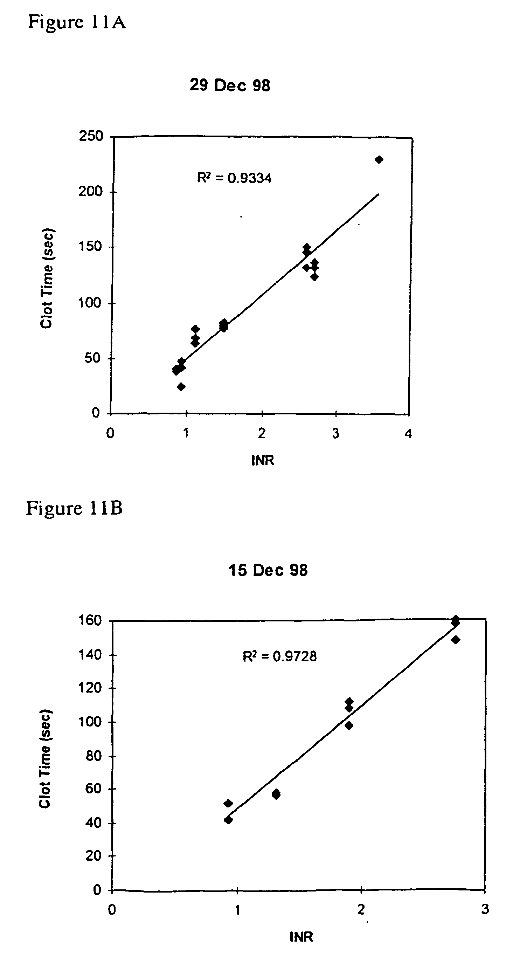

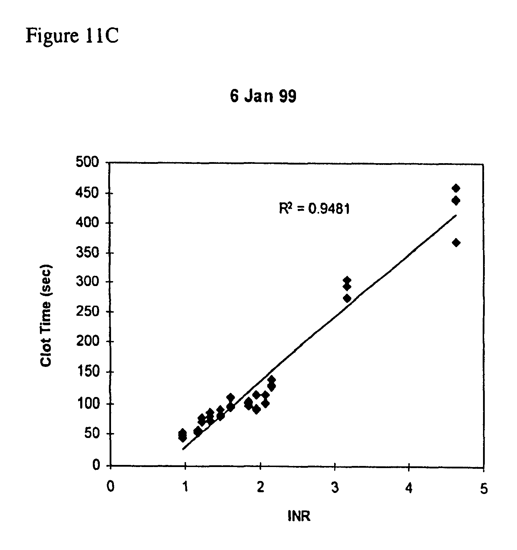

For the purposes of testing the device shown in Figure 8, a 10mL vial of Innovin® (a PT reagent, supplied by Dade International, Inc, Miami, FL, USA) was dissolved in 3.0 mL of 20%w/v mannitol, 0.35% w/v Pluronic F-68. Five μL of the mixture was spread over the reagent area (the wide part of the channel, as defined by the spacer) and dried at a temperature of 50 ° C in a turbo oven (American Scientific products Model Dk-62).
All measurements were then performed at room temperature (23-26 °C). Citrated whole blood samples were obtained from a clinic. The INR value of all specimens was determined with an MLA-1000 apparatus (Medical Laboratory Automation, Inc., Pleasantville, NY, USA), which was used as the reference method.
Amperometric determination of the clotting time with the sensor strip was then performed by connecting electrodes **156** in the sensor to a Wavetek model 130-s-396 function generator, which was adjusted to deliver a 3.5Volts, 10,000Hz square wave AC. The current flowing through the circuit was measured with a data logging Thurlby Thandar Instruments (Huntington, Cambridgeshire, UK) model 1705 multimeter. A schematic drawing of the circuit is depicted in Figure 9, with electrodes **156** connected to an alternating voltage source **162** and to a microoampermeter **164.** Micro-ampermeter **164** was in turn connected to a recording computer **166.**
To perform a coagulation time measurement, voltage source **162** and to microoampermeter **164** were activated so that 0.5Hz data logging started on microoampermeter **164.** Immediately afterward, a 15 μL specimen was applied to the reagent area. Logging of the current measurement continued for 200 seconds. Thereafter, the data was transferred through a serial connection to recording computer **166** for plotting the current vs time transient. Examples of transients for different INR values are presented in Figures 10A and 10B. The coagulation time of the specimen was determined according to the time point at which the current stopped rising. Figures 11A-11C depict the correlation between the clotting time values, as obtained with the sensor strip of the invention, and the INR values, as determined by the reference method.
The values for the prothrombin time were plotted against the INR values determined by the MLA-1000 apparatus. It is clear that the sensor strip of the present invention demonstrated a linear correlation between the measured clotting time and the reference INR value.

Section 3: Detection of a Receptor/Ligand Interaction

An additional preferred embodiment of the device of the present invention could measure a receptor/ligand reaction as follows. A clotting factor would be attached to a receptor or ligand to form a combined clotting factor-acceptor. Hereinafter, the term "acceptor" refers to either a receptor or a ligand. Examples of acceptors include but are not limited to antigen, antibody, nucleic acid probe and lectin. The clotting factor-acceptor complex would then be placed relative to the blood sample such that the reactivity of the clotting factor would then be modulated by the recognition reaction between the acceptor and the corresponding ligand or receptor, respectively. Preferably, the calculation of the alteration in blood clotting time would be determined by comparing the clotting time required for a reference portion of the blood sample which was exposed to the acceptor/coagulation factor combination, to the time required for a second portion which was not exposed to the combination.

For example, if the acceptor was an antigen and the clotting factor was an anti-coagulant, the anti-coagulant would be hidden from the blood sample substantially only if the blood sample contained an antibody which recognized the antigen. Such hiding would decrease the time required for clotting of the blood. The degree to which the blood clotting time decreased could be used to quantitate the amount of antibody present in the blood sample. This preferred embodiment of the device would still measure the clotting of blood according to the amount of current passing through the sample, either when measured after a fixed period of time has elapsed, or by determining the elapsed time required for the maximum measured current to be reached.

The same principle for detection of receptor-ligand reaction can be realized through other viscosity modulating systems, which are well known in the art. Such systems include: dextran and dextranase, DNA and DNase.

It will be appreciated that the above descriptions are intended only to serve as examples, and that many other embodiments are possible within the scope of the present invention as set out in the appended claims.

**Claims**

**1.** A method for measuring a clotting time of a sample of blood from a patient, the method comprising:

(a) providing a disposable sensor card (154) for receiving a blood sample at an end of said sensor, the sensor (12) comprising:

(i) a test chamber (16) with a capillary structure arranged to induce a capillary force on said blood sample to draw a test portion of the blood sample from said end of the sensor into said test chamber;
(ii) a pair of electrodes (18; 20, 22, 156) disposed on the test chamber, such that the test portion of blood is able to contact said pair of electrodes;

(b) placing a blood sample at the end of the sensor such that said capillary force draws said test portion into the test chamber;
(c) providing a voltage generator (126) in electrical contact with the electrodes such that when the test portion is drawn into the test chamber, a first electrical current is passed between said pair of electrodes;
(d) measuring said first electrical current;
(e) using the first electrical current to determine the clotting time.

**2.** A method according to claim 1 wherein the disposable sensor further comprises:

(iii) a reference chamber and a capillary structure arranged to induce a capillary force on the blood sample to draw a reference portion of the blood sample from the end of the sensor into the reference chamber;
(iv) a pair of electrodes disposed on the reference chamber, such that the reference portion of blood is able to contact said pair of electrodes,

the method further comprising:

providing a voltage generator in electrical contact with the electrodes of the reference chamber such that when the reference portion is drawn into the reference chamber, an electrical current is passed between the reference chamber pair of electrodes,
measuring the electrical current passing between the reference chamber pair of electrodes,
using the measured first electrical current and the measured current passing between the reference chamber pair of electrodes to calculate the clotting time.

3. The method of claim 2, wherein calculation of the clotting time is performed by a processor receiving a test signal from the test chamber and a reference signal from the reference chamber, wherein the test and reference signals are used by the processor to calculate the clotting time.

4. A method according to any one of claims 1 to 3 wherein the amplitude of the first electrical current and/or electrical current passing between the reference chamber pair of electrodes is measured after a predetermined period of time from the time at which the sensor is placed in electrical contact with the voltage generator.

5. A method according to any one of claims 1 to 3 wherein the clotting time is determined by measuring the current after a fixed time period.

6. A method according to any one of claims 1 to 3 wherein the clotting time is determined by measuring the period of time required for the amount of current to reach a predetermined level.

7. A method according to any one of claims 1 to 6 wherein the clotting time is prothrombin time or partial prothrombin time.

8. A method according to any one of claims 1 to 7 wherein the step of placing the blood sample at the end of the sensor is performed by the patient.

9. A kit for measuring a clotting time of a sample of blood from a patient, comprising:

   (a) a disposable sensor card (154) for receiving the blood sample at an end of said sensor, the sensor (12) comprising:

      (i) a test chamber (16) with a capillary structure arranged to induce a capillary force on said blood sample to draw a test portion of the blood sample from said end of the sensor into the test chamber;
      (ii) a pair of electrodes (18; 20, 22; 156) disposed on the test chamber, such that the test portion of blood is able to contact said pair of electrodes;

   (b) a voltage generator (126) for generating a voltage, said voltage generator being in electrical contact with the electrodes such that when the test portion is drawn into the test chamber a first electrical current is passed between said pair of electrodes;
   (c) a measurer (129; 152) for measuring said first electrical current, such that the first electrical current is a measured current;
   (d) a processor (32) for determining the clotting time according to the measured current.

10. A kit according to claim 9 wherein the disposable sensor further comprises:

   (iii) a reference chamber and a capillary structure arranged to induce a capillary force on the blood sample to draw a reference portion of the blood sample from said end of said sensor into the reference chamber;
   (iv) a pair of electrodes disposed on the reference chamber, such that the reference portion of blood is able to contact said pair of electrodes,

   wherein the voltage generator is in electrical contact with the electrodes of the reference chamber such that when the reference portion is drawn into the reference chamber, an electrical current is passed between the reference chamber pair of electrodes, and the measurer is arranged to measure the electrical current passing between the reference chamber pair of electrodes, and the processor is arranged to receive a signal from the test chamber electrodes and a signal from the reference chamber electrodes to calculate the clotting time.

11. A kit according to claim 9 or 10 wherein the processor include instructions for calculating the clotting time from the measured first electrical current and/or electrical current passing between the reference chamber pair of electrodes.

12. A kit according to any one of claims 9 to 11, wherein said capillary structure comprises a capillary depth of said test chamber, or a narrow channel shape of said test chamber.

13. A kit according to any one of claims 9 to 12, wherein the test chamber features a single capillary channel.

14. A kit according to any one of claims 9 to 13, wherein at least one dried coagulant agent is provided in the test chamber and/or reference chamber to contact the test and/or reference portion respectively, said coagulant agent altering a clotting property of the test and/or reference portion.

15. A kit according to claim 14 wherein the disposable sensor is a plastic card and wherein the electrodes are screen printed onto the card.

16. A kit according to any one of claims 9 to 15 wherein the clotting time is prothrombin time or partial prothrombin time.

**Patentansprüche**

1. Verfahren zur Messung der Blutgerinnungszeit einer Blutprobe eines Patienten, wobei das Verfahren umfasst:

   (a) Bereitstellen einer Einweg-Sensorkarte (154) zur Aufnahme einer Blutprobe an einem Ende des Sensors, wobei der Sensor (12) umfasst:

   (i) eine Testkammer (16) mit einer Kapillarstruktur, die so angeordnet ist, dass sie eine Kapillarkraft auf die Blutprobe ausübt, um einen Testteil der Blutprobe von dem Ende des Sensors in die Testkammer zu ziehen;
   (ii) ein Paar von Elektroden (18, 20, 22, 156), die in der Testkammer angeordnet sind, so dass der Testteil des Bluts in Kontakt mit dem Paar von Elektroden kommen kann;

   (b) Platzieren einer Blutprobe am Ende des Sensors, so dass die Kapillarkraft den Testteil in die Testkammer zieht;
   (c) Bereitstellen eines Spannungsgenerators (126) in elektrischem Kontakt mit den Elektroden, so dass, wenn der Testteil in die Testkammer gezogen wird, ein erster elektrischer Strom zwischen dem Paar von Elektroden läuft;
   (d) Messen des ersten elektrischen Stroms;
   (e) Verwenden des ersten elektrischen Stroms, um die Blutgerinnungszeit zu bestimmen.

2. Verfahren gemäß Anspruch 1, worin der Einweg-Sensor ferner umfasst:

   (iii) eine Referenzkammer und eine Kapillarstruktur, die angeordnet ist, um eine Kapillarkraft an der Probe zu induzieren, um einen Referenzteil der Blutprobe von dem Ende des Sensors in die Referenzkammer zu ziehen;
   (iv) ein Paar von Elektroden, die in der Referenzkammer angeordnet sind, so dass der Referenzteil des Bluts in Kontakt mit dem Paar von Elektroden kommen kann,

   wobei das Verfahren ferner umfasst:

   Bereitstellen eines Spannungsgenerators in elektrischem Kontakt mit den Elektroden der Referenzkammer, so dass, wenn der Referenzteil in die Referenzkammer gezogen wird, ein elektrischer Strom zwischen dem Paar von Elektroden in der Referenzkammer läuft,
   Messen des elektrischen Stroms, der zwischen dem Paar von Elektroden in der Referenzkammer läuft,
   Verwenden des gemessenen ersten elektrischen Stroms und des gemessenen Stroms, der zwischen dem Paar von Elektroden in der Referenzkammer läuft, um die Blutgerinnungszeit zu berechnen.

3. Verfahren gemäß Anspruch 2, worin die Berechnung der Blutgerinnungszeit mit einem Prozessor durchgeführt wird, der ein Testsignal von der Testkammer und ein Referenzsignal von der Referenzkammer erhält, worin die Test- und Referenzsignale von dem Prozessor verwendet werden, um die Blutgerinnungszeit zu berechnen.

4. Verfahren gemäß irgendeinem der Ansprüche 1 bis 3, worin die Amplitude des ersten elektrischen Stroms und/oder des elektrischen Stroms, der zwischen dem Paar von Elektroden in der Referenzkammer läuft, nach einer vorbestimmten Zeitspanne von der Zeit, bei dem der Sensor in elektrischem kontakt mit dem Spannungsgenerator platziert wird, gemessen wird/werden.

5. Verfahren gemäß irgendeinem der Ansprüche 1 bis 3, worin die Blutgerinnungszeit durch Messen des Stroms nach einer festgelegten Zeitspanne bestimmt wird.

6. Verfahren gemäß irgendeinem der Ansprüche 1 bis 3, worin die Blutgerinnungszeit durch Messen der Zeitspanne bestimmt wird, die erforderlich ist, damit die Strommenge ein vorbestimmtes Niveau erreicht.

7. Verfahren gemäß irgendeinem der Ansprüche 1 bis 6, worin die Blutgerinnungszeit die Prothrombin-Zeit oder partielle Prothrombin-Zeit ist.

8. Verfahren gemäß irgendeinem der Ansprüche 1 bis 7, worin der Schritt zum Platzieren der Blutprobe an dem Ende des Sensors vom Patienten durchgeführt wird.

9. Kit zum Messen der Blutgerinnungszeit einer Blutprobe von einem Patienten, umfassend:

   (a) eine Einweg-Sensorkarte (154) zum Aufnehmen der Blutprobe an einem Ende des Sensors, wobei der Sensor (12) umfasst:

   (i) eine Testkammer (16) mit einer Kapillarstruktur, die so angeordnet ist, dass sie eine Kapillarkraft auf die Blutprobe ausübt, um einen Testteil der Blutprobe von dem Ende des Sensors in die Testkammer zu ziehen;
   (ii) ein Paar von Elektroden (18, 20, 22, 156), die in der Testkammer angeordnet sind, so dass der Testteil des Bluts in Kontakt mit dem Paar von Elektroden kommen kann;

   (b) einen Spannungsgenerator (126) zum Generieren einer Spannung, wobei der Spannungsgenerator in Kontakt mit den Elektroden steht, so dass, wenn der Testteil in die Testkammer gezogen ist, ein erster elektrischer Strom zwischen dem Paar von Elektroden läuft;
   (c) eine Messeinrichtung (129; 152) zum Messen des ersten elektrischen Stroms, so dass der erste elektrische Strom ein gemessener Strom ist;
   (d) einen Prozessor (32) zum Bestimmen der Blutgerinnungszeit gemäß dem gemessenen Strom.

10. Kit gemäß Anspruch 9, worin der Einweg-Sensor ferner umfasst:

   (iii) eine Referenzkammer und eine Kapillarstruktur, die angeordnet ist, um eine Kapillarkraft an der Probe zu induzieren, um einen Referenzteil von der Blutprobe von dem Ende des Sensors in die Referenzkammer zu ziehen;
   (iv) ein Paar von Elektroden, die in der Referenzkammer angeordnet sind, so dass der Referenzteil des Bluts in Kontakt mit dem Paar von Elektroden kommen kann,

   worin der Spannungsgenerator in elektrischem Kontakt mit den Elektroden der Referenzkammer steht, so dass, wenn der Referenzteil in die Referenzkammer gezogen ist, ein elektrischer Strom zwischen dem Paar von Elektroden in der Referenzkammer läuft, und die Messvorrichtung angeordnet ist, um den elektrischen Strom zu messen, der zwischen dem Paar von Elektroden in der Referenzkammer läuft, und der Prozessor angeordnet ist, um ein Signal von den Testkammer-Elektroden und ein Signal von den Referenzkammer-Elektroden zu erhalten, um die Blutgerinnungszeit zu berechnen.

11. Kit gemäß Ansprüche 9 oder 10, worin der Prozessor Befehle zum Berechnen der Blutgerinnungszeit aus dem gemessenen ersten elektrischen Strom und/oder dem elektrischen Strom, der zwischen dem Paar von Elektroden in der Referenzkammer läuft, umfasst.

12. Kit gemäß irgendeinem der Ansprüche 9 bis 11, worin die Kapillarstruktur eine Kapillartiefe der Testkammer oder eine enge Kanalform der Testkammer umfasst.

13. Kit gemäß irgendeinem der Ansprüche 9 bis 12, worin die Testkammer einen einzelnen Kapillarkanal aufweist.

14. Kit gemäß irgendeinem der Ansprüche 9 bis 13, worin zumindest ein getrocknetes Gerinnungsmittel in der Testkammer und/oder der Referenzkammer vorgesehen ist, um in Kontakt mit dem Test- und/oder dem Referenzteil zu kommen, wobei das Gerinnungsmittel die Gerinnungseigenschaften des Test- und/oder Referenzteils verändert.

15. Kit gemäß Anspruch 14, worin der Einweg-Sensor eine Kunststoffkarte ist und worin die Elektroden mittels Siebdruck auf die Karte aufgebracht sind.

16. Kit gemäß irgendeinem der Ansprüche 9 bis 15, worin die Blutgerinnungszeit die Prothrombin-Zeit oder die partiell Prothrombin-Zeit ist.

**Revendications**

1. Un procédé pour mesurer un temps de coagulation d'un échantillon de sang provenant d'un patient, le procédé comprenant :

   (a) la fourniture d'une carte (154) à capteur jetable pour la réception d'un échantillon de sang à une extrémité dudit capteur, le capteur (12) comprenant :

      (i) une chambre de test (16) avec une structure capillaire agencée pour induire une force capillaire sur ledit échantillon de sang pour tirer une portion de test de l'échantillon de sang depuis ladite extrémité du capteur à l'intérieur de ladite chambre de test ;
      (ii) une paire d'électrodes (18, 20, 22, 156) disposées sur la chambre de test, de manière à ce que la portion de test de sang est capable de toucher ladite paire d'électrodes ;

   (b) le placement d'un échantillon de sang à l'extrémité du capteur de manière à ce que ladite force capillaire tire ladite portion de test à l'intérieur de la chambre de test ;
   (c) la fourniture d'un générateur de tension (126) en contact électrique avec les électrodes de manière à ce que quand la portion de test est tirée à l'intérieur de la chambre de test, un premier courant électrique est passé entre ladite paire d'électrodes ;
   (d) la mesure dudit premier courant électrique ;
   (e) l'utilisation du premier courant électrique pour déterminer le temps de coagulation.

2. Le procédé selon la revendication 1 dans lequel le capteur jetable comprend en outre :

   (iii) une chambre de référence et une structure capillaire prévue pour induire une force capillaire sur l'échantillon de sang pour tirer une portion de référence de l'échantillon de sang depuis l'extrémité du capteur à l'intérieur de la chambre de référence ;
   (iv) une paire d'électrodes disposées sur la chambre de référence, de manière à ce que la portion de référence de sang est capable de toucher ladite paire d'électrodes,

   le procédé comprenant en outre :

   la fourniture d'un générateur de tension en contact électrique avec les électrodes de la chambre de référence de manière à ce que quand la portion de référence est tirée à l'intérieur de la chambre de référence, un courant électrique est passé entre la paire d'électrodes de la chambre de référence,
   la mesure du courant électrique passant entre la paire d'électrodes de la chambre de référence,
   l'utilisation du premier courant électrique mesuré et du courant passant entre la paire d'électrodes de la chambre de référence mesuré pour calculer le temps de coagulation.

3. Le procédé selon la revendication 2, dans lequel le calcul du temps de coagulation est mis en oeuvre par un processeur recevant un signal de test provenant de la chambre de test et un signal de référence provenant de la chambre de référence, les signaux de test et de référence étant utilisés par le processeur pour calculer le temps de coagulation.

4. Le procédé selon l'une quelconque des revendications 1 à 3 dans lequel l'amplitude du premier courant électrique et/ou courant électrique passant entre la paire d'électrodes de la chambre de référence est mesurée après une période de temps prédéterminée à partir du moment auquel le capteur est placé en contact électrique avec le générateur de tension.

5. Le procédé selon l'une quelconque des revendications 1 à 3 dans lequel le temps de coagulation est déterminé en mesurant le courant après une période de temps fixée.

6. Le procédé selon l'une quelconque des revendications 1 à 3 dans lequel le temps de coagulation est déterminé en mesurant la période de temps requise pour que la quantité de courant atteigne un niveau prédéterminé.

7. Le procédé selon l'une quelconque des revendications 1 à 6 dans lequel le temps de coagulation est un temps de Quick ou un temps de Quick partiel.

**8.** Le procédé selon l'une quelconque des revendications 1 à 7 dans lequel l'étape de placement de l'échantillon de sang à l'extrémité du capteur est mise en oeuvre par le patient.

**9.** Un prêt-à-monter pour mesurer un temps de coagulation d'un échantillon de sang provenant d'un patient, comprenant :

(a) une carte (154) à capteur jetable pour la réception de l'échantillon de sang à une extrémité dudit capteur, le capteur (12) comprenant :

(i) une chambre de test (16) avec une structure capillaire agencée pour induire une force capillaire sur ledit échantillon de sang pour tirer une portion de test de l'échantillon de sang depuis ladite extrémité du capteur à l'intérieur de ladite chambre de test ;
(ii) une paire d'électrodes (18, 20, 22, 156) disposées sur la chambre de test, de manière à ce que la portion de test de sang est capable de toucher ladite paire d'électrodes ;

(b) un générateur de tension (126) pour générer une tension, ledit générateur de tension étant en contact électrique avec les électrodes de manière à ce que quand la portion de test est tirée à l'intérieur de la chambre de test un premier courant électrique est passé entre ladite paire d'électrodes ;
(c) un mesureur (129, 152) pour mesurer ledit premier courant électrique, de manière à ce que le premier courant électrique est un courant mesuré ;
(d) un processeur (32) pour déterminer le temps de coagulation en fonction du courant mesuré.

**10.** Le prêt-à-monter selon la revendication 9 dans lequel le capteur jetable comprend en outre :

(iii) une chambre de référence et une structure capillaire prévue pour induire une force capillaire sur l'échantillon de sang pour tirer une portion de référence de l'échantillon de sang depuis ladite extrémité dudit capteur à l'intérieur de la chambre de référence ;
(iv) une paire d'électrodes disposées sur la chambre de référence, de manière à ce que la portion de référence de sang est capable de toucher ladite paire d'électrodes,

dans lequel le générateur de tension est en contact électrique avec les électrodes de la chambre de référence de manière à ce que quand la portion de référence est tirée à l'intérieur de la chambre de référence, un courant électrique est passé entre la paire d'électrodes de la chambre de référence, et le mesureur est prévu pour mesurer le courant électrique passant entre la paire d'électrodes de la chambre de référence, et le processeur est prévu pour recevoir un signal provenant des électrodes de la chambre de test et un signal provenant des électrodes de la chambre de référence pour calculer le temps de coagulation.

**11.** Le prêt-à-monter selon la revendication 9 ou 10 dans lequel le processeur comporte des instructions pour calculer le temps de coagulation à partir du premier courant électrique mesurée et/ou courant électrique passant entre la paire d'électrodes de la chambre de référence.

**12.** Le prêt-à-monter selon l'une quelconque des revendications 9 à 11, dans lequel ladite structure capillaire comprend une profondeur capillaire de ladite chambre de test, ou une forme de canal étroit de ladite chambre de test.

**13.** Le prêt-à-monter selon l'une quelconque des revendications 9 à 12, dans lequel la chambre de test présente un seul canal capillaire.

**14.** Le prêt-à-monter selon l'une quelconque des revendications 9 à 13, dans lequel au moins un agent coagulant séché est fourni dans la chambre de test et/ou la chambre de référence pour toucher respectivement la portion de test et/ou de référence, ledit agent coagulant modifiant une propriété de coagulation de la portion de test et/ou de référence.

**15.** Le prêt-à-monter selon la revendication 14 dans lequel le capteur jetable est une carte plastique et dans lequel les électrodes sont imprimées en sérigraphie au dessus de la carte.

**16.** Le prêt-à-monter selon l'une quelconque des revendications 9 à 15, dans lequel le temps de coagulation est un temps de Quick ou un temps de Quick partiel.

Figure 1.

| | |
|---|---|
| Connect electrodes to capacitance measurer | Step one |
| Apply sample to chamber | Step two |
| Move liquid | Step three |
| Change capacitance property | Step four |
| Determine viscosity | Step five |

Figure 2A.

Figure 2B

EP 1 064 532 B1

Figure 2C.

Figure 2D.

capacitance measuring circuit (25)

measurer

Converter

Processor

Clock

Screen

Data storage

EP 1 064 532 B1

Figure 3

Figure 4.

```
┌─────────────────┐
│  Supply power to │              Step one
│    electrodes    │
└─────────────────┘
         │
         ▼
┌─────────────────┐
│  Apply sample to │              Step two
│     chamber      │
└─────────────────┘
         │
         ▼
┌─────────────────────┐
│     Move liquid     │
└─────────────────────┘          Step three
         │
         ▼
┌─────────────────┐
│ Change electrical│              Step four
│     property     │
└─────────────────┘
         │
         ▼
┌─────────────────┐
│    Determine     │
│    viscosity     │              Step five
└─────────────────┘
```

Figure 5A.

EP 1 064 532 B1

Figure 5B

EP 1 064 532 B1

Figure 5C.

Figure 6A

Time for max. current

Figure 6B

Current after 37 sec.

Figure 6C

Figure 7A

Figure 7B

Figure 8

Base Card
with
Electrodes

Spacer

Mesh

EP 1 064 532 B1

Figure 9

156

156

Sensor Strip with Electrodes

164

Recording Computer

166

Microampermeter

mA

Alternating Voltage source

162

Figure 10A

**2.08 #1898**

Figure 10B

Figure 11A

**29 Dec 98**

$R^2 = 0.9334$

Figure 11B

**15 Dec 98**

$R^2 = 0.9728$

Figure 11C

6 Jan 99

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 3951606 A
- WO 9600395 A
- US 5039617 A
- US 5197017 A
- US 3699437 A
- US 3674012 A
- US 5491408 A

- DD 237454
- US 5601995 A
- US 3840806 A
- WO 9416095 A
- US 5628961 A
- US 5120420 A
- US 5720733 A

**Non-patent literature cited in the description**

- **A. Ur.** *Nature,* 1970, vol. 226, 269-270